(19)

Europäisches
Patentamt
European
Patent Office
Office européen
des brevets

(11) **EP 3 578 182 A1**

(12) **EUROPEAN PATENT APPLICATION**

(43) Date of publication:
**11.12.2019 Bulletin 2019/50**

(21) Application number: **18176139.6**

(22) Date of filing: **05.06.2018**

(51) Int Cl.:
*A61K 31/53* [(2006.01)]          *A61K 45/06* [(2006.01)]
*A61K 9/00* [(2006.01)]           *A61K 31/555* [(2006.01)]
*A23K 50/60* [(2016.01)]          *A23L 33/10* [(2016.01)]
*A23L 33/165* [(2016.01)]         *A61K 47/60* [(2017.01)]

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Bayer Animal Health GmbH
51373 Leverkusen (DE)**

(72) Inventor: **The designation of the inventor has not
yet been filed**

(74) Representative: **Tier 1 Patents
C/o Bayer Animal Health GmbH
Kaiser-Wilhelm-Allee 20
51373 Leverkusen (DE)**

(54) **FORMULATIONS CONTAINING TRIAZINONES AND IRON WITH A LOW AMOUNT OF FREE
IRON IONS**

(57)    Formulations containing triazinones, selected from toltrazuril, ponazuril and diclazuril, a polynuclear iron(III) polysaccharide complex compound which are suitable for the simultaneous control of coccidiosis and anaemic states in animals and which exhibit a low amount of free iron ions even after storage, while the formulations comprises $Ca^{2+}$ and $Na^+$ in a molar ratio of $Ca^{2+}$:$Na^+$ from $\geq 0$ to $\leq 3$. In one embodiment the triazinone is toltrazuril and the polynuclear iron(III) polysaccharide complex compound is iron(III) dextran glucoheptonate.

Figure 1: free iron measured after 12 months of storage at 30°C according to Example 2

EP 3 578 182 A1

**Description**

The present invention relates to formulations containing triazinones and a polynuclear iron(III) polysaccharide complex compound which are suitable for the simultaneous control of coccidiosis and anaemic states in animals and which exhibit a low amount of free iron ions even after storage.

Economically successful meat production operations are currently distinguished by highly intensive farming, that is to say by the keeping of a large number of animals which are specifically selected in order to optimize the breeding aim. These farms are characterized for example by the use of a great deal of machinery, the additional feeding of food supplements, and the involvement of as little staff as possible. In the case of piglet rearing farms, this means that a large number of sows which are bred for a high number of piglets per litter are kept in suitably large pig houses. The optimization of the feed, and suitable selection in the breeding process, make it possible for the piglets to grow rapidly.

This type of animal keeping is frequently the cause for an increasing number of certain typical diseases and deficiencies. Besides stress, to which in particular intensively kept pigs are very susceptible, such phenomena are, in young pigs, protozoal infections (coccidiosis) and anaemic states, inter alia, both of which already have to be kept under control by the prophylactic use of medicaments.

Coccidiosis are frequently occurring, parasitic infectious diseases in animals. Thus, for example, protozoans of the genera Eimeria, Isospora, Neospora, Sarcosporidia and Toxoplasma cause coccidiosis all over the world. Examples of economically important coccidiosis are: infections of pigs with coccidia of the genus Isospora or of cattle with coccidia of the genus Eimeria. Infections with Isospora suis have only in recent years been recognized as the cause of diarrhea in piglets and studied intensively. As a rule, an infection proceeds from the environment to the piglets, or from piglet to piglet, via oocysts, which contain in each case two sporocysts with in each case two sporozoites. The parasitic stages multiply in the epithelial cells of the small intestine's villi. The clinical picture of the disease includes a necrotic, inflammatory destruction of the gut's epithelial cells with atrophying villi, and, as a result, impaired absorption and digestion. The characteristic of an acute disease is a liquid, whitish to yellow diarrhea, which mostly occurs in week 2 to 3 of life. The weight gain of infected piglets is reduced. Treatment and therapy of the disease are insufficient to date.

Antibiotics are ineffective; while sulphonamides are approved for the treatment of coccidiosis, their effect is questionable, and frequently repeated administrations are in any case unsuitable for practice. Other possible treatments are questionable: the administration of, for example, monensin, amprolium or furazolidon has not been successful in preventing the disease in experimentally infected piglets. In more recent studies, Isospora suis has been identified in up to 92% of all litters in some farms, despite good hygiene. This type of disease is not limited to pigs, but also occurs in many other animal species, for example in poultry production, in calves, lambs or in small animals (rabbits).

An example of a deficiency is iron deficiency in newly-born piglets. Owing to the rapid growth in the first days after birth, the body's iron reserves are rapidly depleted and must be compensated for by external sources. Because of the large number of suckling pigs, this substitution by taking up the sow's milk cannot take place in a sufficient degree. If, moreover, the animals are kept on concrete or plastic, the piglets cannot take up iron compounds by rooting in the ground either. The piglets become anaemic. A clinically significant anaemic state exists when the hemoglobin content of the blood has dropped to less than 80 g/L. The NRC recommendation (National Research Council, Nutrient Requirements of Domestic Animals, No. 2, Nutrient Requirements of Swine, National Academy of Sciences, Washington DC, 1973) specifies 90 g/L as the minimum hemoglobin value at which the piglets grow healthily and show no signs of anaemia. Noticeable symptoms such as weight loss or stunted growth are, however, only observed when the hemoglobin content of the blood has dropped to values of below 80 g/L. Other indicators for the iron supply are the hematocrit and the number of erythrocytes per unit volume. Severe iron deficiency anaemia also leads to the young pigs' death.

Preparations are already available for controlling the above-mentioned diseases and deficiencies.

Coccidiosis can be controlled successfully by administering active ingredients from the triazinone group. To this end, one distinguishes between the triazinediones - with examples of representatives being the active ingredients clazuril, diclazuril, letrazuril - and the triazinetriones with the active ingredients toltrazuril, toltrazuril sulphoxide and ponazuril. Triazines, in particular toltrazuril, ponazuril or diclazuril, and their activity against coccidia are known from a series of publications, see, inter alia, DE-A 27 18 799 and DE-A 24 137 22. WO 99/62519 discloses semisolid aqueous preparations of toltrazuril sulphone (ponazuril). It is also known that it is in particular toltrazuril which is suitable for treating coccidiosis (for example Isospora suis) in pigs. See, for example, also the following publications: Don't forget coccidiosis, update on Isosporosis in piglets. Part I, Pig Progress volume 17, No. 2, 12-14; Mundt., H.-C., A. Daugschies, V. Letkova (2001): be aware of piglet coccidiosis diagnostics. Part II, Pig Progress volume 17, No. 4, 18-20; Mundt, H.-C., G.-P1 Martineau, K. Larsen (2001): control of coccidiosis Part III, Pig Progress volume 17, No. 6, 18-19.

Examples of important products on the market are diclazuril (2,6-dichloro-α-(4-chlorophenyl)-4-(4,5-dihydro-3,5-dioxo-1,2,4-triazin-2(3H)-yl)benzeneacetonitrile; CAS No. 101831-37-2) (CLINACOX™ 0.5%, Janssen Animal Health; VECOXAN™, Biokema SA) for admixture to the feed and toltrazuril (1-methyl-3-[3-methyl-4-[4-[(trifluoromethyl)thio]phenoxy]phenyl]-1,3,5-triazine-2,4,6(1H,3H,5H)trione; CAS No. 69004-03-1). Toltrazuril is available on the market for example as a drinking water formulation for poultry and as an oral suspension formulation for, inter alia, the treatment of suckling pigs. It is recommended to administer, to the piglet, a dose of 20 mg/kg bodyweight on day 3-5 after birth.

The disadvantage in the oral administration of the above-mentioned anticoccidials (sometimes also somewhat inaccurately referred to as coccidiostats) is that it is relatively laborious: the piglets must be caught and the product is administered into the throat with the aid of an applicator or a drench gun. Moreover, the method causes not inconsiderable stress for the piglets.

EP 2 740 469 A1 concerns methods for protecting non-human animals with triazine compounds by intramuscular or subcutaneous injection(s). The concept can be used with various triazines, such as toltrazuril, in different non-human animals such as a porcine, an ovine, a bovine, a canine, a feline, or an avian, for protecting them against infectious diseases, such as protozoan disorders.

EP 2 740 470 A1 relates to relates to intramuscular compositions and methods for treating coccidiosis with a triazine.

For the treatment of iron deficiencies, a group of compounds exists which are applied mainly parenterally and only to a minor extent orally and which consists of of fairly stable compounds of the poly-$\beta$-FeO(OH) type with complex-bound polymeric carbohydrates. Commercial importance has been gained mainly, but not exclusively, by iron(III) dextran (CAS No. 9004-66-4), iron (III) dextran glucoheptonate (Gleptoferron, CAS No. 57680-55-4), iron(III) hydroxide polymaltose (iron(III) hydroxide dextrin; CAS No. 53858-86-9), iron(III) sucrose (iron(III) sucrose, iron(III) "sugar" CAS No. 8047-67-4) and sodium/iron(III) gluconate complex in sucrose solution (CAS No. 34089-81-1).

The literature reveals different names for these compounds. In this context, compounds such as Gleptoferron, iron(III) dextran, iron(III) polymaltose, iron(III) dextrin, iron(III) sucrose, iron(III) gluconate, iron(III) sugar are understood as meaning complexes of the iron(3+) ion with hydroxide ions (OH-), aquo groups ($H_2O$) and oxygen (O), which complexes are present in oligomeric or polymeric form and which are associated, in their coordination sphere, in the form of complexes with one or more of the above-mentioned oligomeric and polymeric carbohydrate compounds. This is why the compounds are also referred to as iron(III) hydroxide polysaccharide or iron(III) oxyhydroxy polysaccharide, where polysaccharide stands for the above-mentioned oligo- and polymeric carbohydrate compounds or their derivatives or, generally, for compounds from the group of the oligomeric or polymeric carbohydrates.

Polynuclear iron(III) complexes of this type are described for example in (D.S. Kudasheva et al., "Structure of Carbohydrate-bound Polynuclear Oxyhydroxide Nanoparticles in Parenteral Formulation", J. Inorg. Biochem. 98 (2004) 1757-1769; I. Erni et al., "Chemical Characterization of Iron(III) Hydroxide-Dextrin Complexes" Arzneim.-Forsch./Drug Res. 34 (II) (1984) 1555-1559; F. Funk et al., "Physical and Chemical Characterization of Therapeutic Iron Containing Materials", Hyperfine Interactions 136 (2001) 73-95; E. London "The Molecular Formula and Proposed Structure of the Iron-Dextran Complex, IMFERON", J. Pharm. Sci. 93 (2004) 1838-1846; A. John "Neue Möglichkeiten der Eisenversorgung neugeborener Ferkel unter Beachtung biochemischer Aspekte" [Novel possibilities of supplying iron to new born piglets, taking account of biochemical aspects], Trächtigkeit und Geburt beim Schwein [Pregnancy and birth in pigs]: 8th Bernburger Biotechnology Workshop, Bernburg (2002) 89-94). Since in many cases the composition of these compounds is not described in quantitative terms, and may also vary within the compounds, depending on the type of preparation, these polynuclear iron(III) polysaccharide compounds are understood as meaning all complexes of the above-described class of compounds which are known to the skilled worker.

One of those iron(III) polysaccharide complex compounds, iron (III) dextran glucoheptonate (Gleptoferron, CAS No. 57680-55-4), has become of particular importance in veterinary field and is marketed, for example, under a variety of tradenames like Gleptosil™, Ursoferran™, Imposil™ or Heptomer™. Unlike with iron(III) dextran, not many structural or physico-chemical investigations have been carried out with this modified iron(III) dextran. The polynuclear poly-$\beta$-FeO(OH) complex core is stabilized by dextran, whose terminal glucose group has been modified by a reaction with cyanide to form glucoheptonate terminal groups.

When the complex is formed and, by proper control of pH and temperature, it precipitates in-situ in form of nano-cyrstalline particles of less than 100nm in dimension in the aqueous solution, therefore forming a true colloidal solution. In the traded products mentioned above, phenol is added for preservation. The mass share of iron in such complex may vary to some extent, but it is usually about 40% . The mass share of the complex in the aqueous colloidal solution may also vary, but it is usually around 50%. There is no more detailed structural investigation available, but in general the structure should be comparable with the findings of London, who investigated the iron(III) dextran complex in detail (E.London; The molecular formula and proposed structure of the iron-dextran complex, Imferon; J. Pharm. Sci 93(7) (2004) 1838-1845). These iron compounds are used almost exclusively in the manufacture of preparations for injection for human and veterinary medicine. In veterinary medicine, additionally, preparations for oral administration are also in use. These complexes are generally distinguished by high stability and differ mainly with regard to their molecular weight, which may vary from 30 kDa up to 400 kDa, and in the strength of the complex binding. In aqueous solution, they are present as colloid dispersions with a particle size of generally 7-35 nm.

Although some products for oral administration are on the market, it is more conventional in pig rearing to administer polynuclear iron(III) complexes intramuscularly by means of an injection. This is carried out as a rule by injecting 100-200 mg of active iron on day 3 after birth. The transport away from the site of injection takes place via the lymphatic system and the cells of the reticulohistiocytary system. The complexes are stored in the liver and the spleen, from where they are liberated as required and metabolized enzymatically. The different metabolism pathways involve serveral transport proteins and reduction/oxidation steps such as and via Mobilferrin ($Fe^{3+}$), Ferroportin ($Fe^{2+}$) and Transferrin ($Fe^{3+}$). $Fe^{3+}$ is ultimately bound to transferrin and transferred to the sites of use in the bone marrow where it is either stored as a Ferritin complex ($Fe^{3+}$) or made available to the iron-containing physiological molecular entities.

The transport capacities of the carrier proteins for free iron(III)- or iron(II)-ions in organisms is limited. Should the concentration of free iron-ions exceed the transport and storage capacity of the organism, unbound $Fe^{2+}$ and $Fe^{3+}$ will bind unspecifically to enzymes and proteins and cause their degeneration, or free radicals will form and cause harmful reactions. Such an overload could be triggered accidentally by an overdosing of a beneficial iron containing drug product or by inherent instability of the iron polysaccharide complex which becomes evident by a high content of "free" or "labile" unbound iron ions or small complex fragments. It is the current opinion that minute amounts of free $Fe^{2+}$ ions are responsible for the formation of free-radical compounds with organic molecules, for example the lipid-peroxide compounds, which are associated with the high potassium content in the blood. Vitamin E acts as a free-radical scavenger and is capable of buffering these harmful reactions in a certain manner, but this frequently exceeds the body's capacities (this is why vitamin E is also added to the oral preparations which have already been mentioned, in which the iron(III) dextran is bound to micro-emulsion droplets).

The art of manufacturing those polynuclear iron(III) polysaccharide complexes and other low molecular weight iron complexes like iron(II) glycinate and others, is therefore to carefully trigger the balance of complex stability and lability. The polynuclear iron(III) polysaccharide complex must be stable enough to avoid a high load of unbound iron ions or freely dissolved small fractions of the complex either originating from an inappropriate manufacturing process or by degradation upon storage over time or at higher temperatures. On the other hand, the polynuclear iron(III) polysaccharide complex must not be too stable, so that the decomposition and disintegration after administration by transport proteins like mobilferrin and others cannot take place properly. The polynuclear iron(III) polysaccharide complexes, namely in the form of nanocrystalline colloidal particles as they are present in the aqueous solutions of marketed drug products, thus can be viewed as a slow release depot entity in the interstitial tissue after injection or in the intestinal mucosa after oral administration.

It is therefore vital to control and reduce the formation of "free" iron entities in iron(III) polyacccharide solutions - and formulations containing those polynuclear iron(III) polysaccharide complexes - upon production and upon storage.

This becomes of special importance when those formulations are presented together with other beneficial agents in combination formulations. The effect of any ingredient which may be added to the colloidal solutions of polynuclear iron(III) polysaccharide complexes needs to be checked for its effect on the stability of the colloidal complex.

It is useful and beneficial to the animal, namely young piglets, if diseases which commonly coincide with iron deficiency are treated with a single administration act, thus reducing the stress to the animals and the handling processes in pig keeping.

Useful classes of beneficial agents which may be co-administered with iron carbohydrate complex solutions in the treatment of piglets and other animals are, for example but not limited to, dietary supplements such as vitamins and mineral supplements, agents with increase the well-being and robustness of the animals, for example immune-stimulants and vaccination, and drug classes like antibiotics, analgesics or anti-coccidiosis drugs. As to that regard, combinations with anti-coccidiosis drugs are of particular relevance.

WO 2008/145281 A2 discloses the simultaneous application of triazines such as toltrazuril, ponazuril or diclazuril and iron compounds in a formulation for controlling coccidia infections and iron deficiencies in animals and humans.

WO 2008/145281 A2 discloses sodium propionate and sodium benzoate as preservatives to be potentially present in the formulations, whereby only those two preservatives are a source of sodium in any of the formulations disclosed therein. Particularly WO 2008/145281 A2 fails to disclose $Na^+$ in the form of any salt other than those two preservatives, is silent on the presence of $Ca^{2+}$ and also does not teach or suggest to have any ratio thereof to achieve an effect.

WO 2014/086960 A1 relates to compositions comprising at least one active ingredient of the family of the triazines combined with another active ingredient and specific compounds that allow the reduction of foam formation. More particularly, WO 2014/086960 A1 teaches that - to avoid excessive formation of foam - aqueous suspensions comprising a triazine, an iron complex, and one or more surfactant(s) having a particularly low HLB value, should be used.

While WO 2014/086960 A1 centers on the addition and effect of certain surfactants, it remains silent on any other effects of constituents that may or may not be in the compositions disclosed.

More specifically, and in the context of the present invention, WO 2014/086960 A1 also exemplifies 14 specific formulations. From those 14 exemplified formulations, those of examples 6 and 7 as well as 9 to 14 comprise sodium chloride and were made subject to foam formation testing.

Furthermore, three individual formulations are mentioned on page 18 of WO 2014/086960 A1 that are not tested for foam formation, are disclosed to comprise sodium chloride.

Considering the Examples 9 to 14 of WO 2014/086960 A1 that comprise sodium chloride, it's apparent that those are comparative examples, as none thereof comprise a surfactant that has a HLB of 8 or below being argued to be causative for the inhibition of foam formation pursuant to the alleged invention of WO 2014/086960 A1.

This is evidenced by the fact that all of the formulations in examples 9 to 14 show excessive foam formation (at least at t = 0 min.).

Considering Examples 6, 7 those formulations both comprise 1% sodium chloride.

The implication of the addition of sodium chloride to the formulations disclosed in WO 2014/086960 A1 remains unclear, particularly in view of (comparative) Examples 9 to 14.

The specific formulations disclosed in of WO 2014/086960 A1 that also comprise sodium chloride are more specifically those listed hereunder.

Example 6 describes a formulation, consisting of 3.5% toltrazuril, 17.8% of iron (as Gleptoferron), 0.1% of sorbitan monooleate, 10% diethylene glycol monoethyl ether, 1% of sodium chloride, and Water ad 100%.

Example 7 describes a formulation, consisting of 3.5% toltrazuril, 19.3% of iron (as Gleptoferron), 0.3% of Propylene Glycol Monolaurate, 0.1% of copolymer Polyoxyl 35 - hydrogenated castor oil, 1% Sodium Chloride, and Water ad 100%.

Example 9 describes a formulation, consisting of 4.2% toltrazuril, 13.5% of iron (as Gleptoferron), 0.1% Sodium Docusate, 0.1% silicone emulsion, 1.5% sodium chloride, and Water ad 100%.

Example 10 describes a formulation, consisting of 4.2% toltrazuril, 13.5% of iron (as Gleptoferron), 0.1% Sodium Docusate, 5% diethylene glycol monoeethyl ether, 0.1% silicone emulsion, 1% sodium chloride, and Water ad 100%.

Example 11 describes a formulation, consisting of 4.2% toltrazuril, 13.5% of iron (as Gleptoferron), 0.6% Phenole, 0.2% Sodium Docusate, 0.1% simethicone emulsion USP 30%, 0.5% colloidal silicone dioxide, 1% povidone, 0.5% sodium chloride, and Water ad 100%.

Example 12 describes a formulation, consisting of 4.2% toltrazuril, 13.5% of iron (as Gleptoferron), 0.6% Phenole, 0.2% Sodium Docusate, 0.1% simethicone emulsion USP 30%, 0.5% colloidal silicone dioxide, 1% povidone, 1% sodium chloride, and Water ad 100%.

Example 13 describes a formulation, consisting of 4.2% toltrazuril, 13.5% of iron (as Gleptoferron), 0.6% Phenole, 0.1% Sodium Docusate, 0.1% simethicone emulsion USP 30%, 0.5% colloidal silicone dioxide, 1% povidone, 1% sodium chloride, and Water ad 100%.

Example 14 describes a formulation, consisting of 2.9% toltrazuril, 18.8% of iron (as Gleptoferron), 0.6% Phenole, 0.1% Sodium Docusate, 0.1% simethicone emulsion USP 30%, 0.5% colloidal silicone dioxide, 1% povidone, 1% sodium chloride, and Water ad 100%.

The three explicitly mentioned formulations given on page 18 of WO 2014/086960 A1 consist of 2.9 % toltrazuril, 18.8 % of iron (as Gleptoferron), 0.3 % of sorbitan monooleate, 0.1 % of copolymer 15 Polyoxyl 35 - hydrogenated castor oil, 1.5% of sodium chloride and water, or 4.2 % toltrazuril, 13.5 % of iron (as Gleptoferron), 0.3 % of sorbitan monooleate, 0.1 % of copolymer Polyoxyl 35 - hydrogenated castor oil, 1.5% of sodium chloride and water, or 4.2 % toltrazuril, 13.5 % of iron (as Gleptoferron), 1 % of sodium chloride, 1 % of polyvinylpyrrolidone, 0.6% of phenol, 0.5% of colloidal silicon dioxide, 0.1 % of sodium docusate, 0 .1 % of simethicon emulsion and water.

WO 2014/086960 A1 generically discloses that any of the formulations described are suitable to treat animals against coccidiosis, but fails to disclose that simultaneous treatment of coccidiosis and anemia is pursued and further fails to disclose with regard to those specific formulations mentioned in Examples 6, 7 and 9 to 14 and on page 18 that these may be of any advantage other than potentially inhibiting foam formation. There is nothing in WO 2014/086960 A1 that would imply stability aspects of the formulations related to any of the substances comprised. WO 2014/086960 A1 also fails to disclose any ratio of $Ca^{2+}:Na^+$ or the presence and influence of those ions.

In some marketed products for the treatment of iron deficiency in piglets (for example, FeVit™) iron(III)dextran complex solutions are co-formulated with vitamins.

Other useful combinations are those with mineral supplements. Fast growing piglets may also develop signs of deficiencies regarding mineral salts, for examples hypocalcaemia. Marketed products for injection like Theracalcium™ or Procal™ contain $Ca^{2+}$ gluconate and $Ca^{2+}$ glucoheptonate and are authorized for the treatment of young piglets suffering from $Ca^{2+}$ shortages. Other $Ca^{2+}$ salts may also be used for the treatment, i.e. $Ca^{2+}$ glycerophosphate. The treatment of those deficiencies does often include co-administration of $Mg^{2+}$ salts like $Mg^{2+}$ hypophosphite (Calciject 20CM™).

Although the scientific opinion of the past taught that there may be adverse effect like a noticeable reduction of the iron uptake when the iron compounds are co-administered with $Ca^{2+}$ salts, recent research revealed that this adverse effect is minor or temporary and co-administration of $Ca^{2+}$ and iron compounds may indeed be beneficial. (B.Lönnerdal "Calcium and Iron Adsorption - Mechanisms and Public Health Relevance", Int. J. Vitam. Nutr. Res. 80 (4-5) (2010, 293-299; M.Miranda et al. "Reducing Iron Deficiency Anemia in Bolivian School Children: Calcium and Iron Combined Versus Iron Supplementation Alone", Nutrition 30 (2014) 771-775). As a consequence, combination of $Ca^{2+}$ compounds and iron compounds in single treatments was recommended.

As a matter of example, US2017/0202802A1 reveals an oral combination treatment (capsules for use in humans) of vitamins and iron compounds. The iron compound is provided as iron polysaccharide complex, the vitamins may contain Vitamin B9 in the form of folic acid and L-methylfolate calcium, the Vitamin C may be presented, for example, as calcium ascorbate.

Recently, liquid combination products of iron compounds (Ferrous Asparto-Glycinate) and L-Methylfolate Calcium have been granted permission for manufacture and marketing (Recommendations of the SEC (Oncology&Haematology), 60th meeting, New Delhi, 2017-09-12; for example: I FAG Syrup™ by Akuementis Healthcare Ltd).

CN102835553 discloses the combined administration of calcium gluconate and iron dextran, but lacking triazinones in the form of a premixed feed to treat livestock.

In addition to adding mineral salts to formulations to treat conditional deficiencies in animals, the addition of salt to formulations containing iron compounds, specifically iron polysaccharide complexes, may become necessary for technological reasons: Tonicity adjustment of injectable formulations, control of flocculation in pharmaceutical suspension formulations and freeze point depression in liquid formulations are examples of those needs. Mineral salts as well as organic salts of alkali metals, alkaline earth metals and earth metals may be used for those purposes. However, in all these cases, the effect of added excipients or active pharmaceutical ingredients on the stability of formulations containing iron compounds, namely iron polysaccharide complexes, must be carefully investigated. The assay of free iron ions and labile iron complex fragments is used as the main indicator of the stability of the iron polysaccharide complexes.

Hence, depending on the initial stability of an polynuclear iron(III) polysaccharide complex, storage conditions and the presence of formulation additives, some degradation might occur and free or labile iron might be released. It is therefore important to keep the concentration of free or labile iron in stored injection formulations low in order to avoid the toxic side effects of iron(III) and, after metabolic reduction, iron(II) ions.

The present invention has the object of providing such a storage-stability improved formulation.

It has surprisingly been found that certain ratios of $Ca^{2+}$ to $Na^+$ in colloidal solutions of polynuclear iron(III)polysaccharide complex compounds, formulated as such or in combination with triazinones, are able to stabilize the solutions. This is particularly surprising, as it was also found that the respective individual ions may not or negatively influence and destabilize such polynuclear iron(III)polysaccharide complex compounds.

If formulations according to the present invention that have a specific $Ca^{2+}$ to $Na^+$ ratio are prepared, those formulations have less (harmful) free iron either directly or at least upon storage compared to polynuclear iron(III)polysaccharide complex solutions that fail to have such $Ca^{2+}$ to $Na^+$ ratio.

Accordingly, a formulation comprising a triazinone selected from the list consisting of toltrazuril, ponazuril and diclazuril, a polynuclear iron(III)polysaccharide complex compound, and a molar ratio of $Ca^{2+}$ to $Na^+$ from $\geq 0$ to $\leq 3$, with the exception of sodium propionate and/or sodium benzoate being the sole source of $Na^+$ in the formulation when the molar ratio of $Ca^{2+}$ to $Na^+$ is 0, is a first aspect of the present invention.

In a second aspect of the present invention, a formulation comprising a triazinone selected from the list consisting of toltrazuril, ponazuril and diclazuril, a polynuclear iron(III)polysaccharide complex compound, and a molar ratio of $Ca^{2+}$ to $Na^+$ from $\geq 0$ to $\leq 3$ except for the formulations as to Example 6 and 7 of WO 2014/086960 A1 (as set forth above) is provided.

In a third aspect of the present invention, a formulation comprising a triazinone selected from the list consisting of toltrazuril, ponazuril and diclazuril, a polynuclear iron(III)polysaccharide complex compound, and a molar ratio of $Ca^{2+}$ to $Na^+$ from $\geq 0$ to $\leq 3$ except for the formulations as to Example 9 to 14 of WO 2014/086960 A1 (as set forth above) is provided.

In a fourth aspect of the present invention, a formulation comprising a triazinone selected from the list consisting of toltrazuril, ponazuril and diclazuril, a polynuclear iron(III)polysaccharide complex compound, and a molar ratio of $Ca^{2+}$ to $Na^+$ from $\geq 0$ to $\leq 3$ except for the formulations selected from those consisting of either 1) 2.9 % toltrazuril, 18.8 % of iron (as Gleptoferron), 0.3 % of sorbitan monooleate, 0.1 % of copolymer 15 Polyoxyl 35 - hydrogenated castor oil, 1.5% of sodium chloride and water, 2) 4.2 % toltrazuril, 13.5 % of iron (as Gleptoferron), 0.3 % of sorbitan monooleate, 0.1 % of copolymer Polyoxyl 35 - hydrogenated castor oil, 1.5% of sodium chloride and water, and 3) 4.2 % toltrazuril, 13.5 % of iron (as Gleptoferron), 1 % of sodium chloride, 1 % of polyvinylpyrrolidone, 0.6% of phenol, 0.5% of colloidal silicon dioxide, 0.1 % of sodium docusate, 0.1 % of simethicon emulsion and water (as given on page 18 of WO 2014/086960 A1) is provided.

Any of the just above referred to four aspects of the present invention may be combined, also resulting in potential fifth aspect of the present invention that is a formulation comprising a triazinone selected from the list consisting of toltrazuril, ponazuril and diclazuril, a polynuclear iron(III)polysaccharide complex compound, and a molar ratio of $Ca^{2+}$ to $Na^+$ from $\geq 0$ to $\leq 3$, with the exception of $Na^+$ not being present as either sodium propionate and/or sodium benzoate and except for the formulations as to Example 6, 7, 9 to 14 of WO 2014/086960 A1 (as set forth above) and except for the formulations selected from those consisting of either 1) 2.9 % toltrazuril, 18.8 % of iron (as Gleptoferron), 0.3 % of sorbitan monooleate, 0.1 % of copolymer 15 Polyoxyl 35 - hydrogenated castor oil, 1.5% of sodium chloride and water, 2) 4.2 % toltrazuril, 13.5 % of iron (as Gleptoferron), 0.3 % of sorbitan monooleate, 0.1 % of copolymer Polyoxyl 35 - hydrogenated castor oil, 1.5% of sodium chloride and water, and 3) 4.2 % toltrazuril, 13.5 % of iron (as Gleptoferron), 1 % of sodium chloride, 1 % of polyvinylpyrrolidone, 0.6% of phenol, 0.5% of colloidal silicon dioxide, 0.1 % of sodium docusate, 0 .1 % of simethicon emulsion and water (as given on page 18 of WO 2014/086960 A1).

In a preferred embodiment of the present invention according to either of the five aspects outlined just above, a further exception is made regarding a formulation consisting of 5% toltrazuril, 22.8% Iron in the form of Fe(III)dextrane, 0.2% sodium proprionate, 0.2% sodium benzoate, 0.25% sodium docusate, 0.05% simethicone emulsion USP 30%, 10.5% propylene glycole, 1.03% citric acid, 0.15% bentonite and water ad. 100%.

In a preferred embodiment of the aforementioned aspects of the present invention, a molar ratio of $Ca^{2+}$ to $Na^+$ from $\geq 0$ to $\leq 1$ is present in the formulation. In any of the above referred to aspects of the present invention a molar ratio of $Ca^{2+}$ to $Na^+$ from $\geq 0$ to $\leq 0.8$ is more preferred, even more preferred is a ratio of $Ca^{z+}$ to $Na^+$ from $\geq 0$ to $\leq 0.5$.

In an also preferred embodiment of the aforementioned aspects of the present invention, a molar ratio of $Ca^{2+}$ to $Na^+$ from $> 0$ to $\leq 3$ is present in the formulation. In such an embodiment of any of the above referred to aspects of the present invention a molar ratio of $Ca^{2+}$ to $Na^+$ from $> 0$ to $\leq 1$ is more preferred, even more preferred is a ratio of $Ca^{2+}$ to $Na^+$ from $> 0$ to $\leq 0.8$, and much more preferred is a ratio of $Ca^{z+}$ to $Na^+$ from $> 0$ to $\leq 0.5$.

According to the present invention the molar ratio of $Ca^{2+}$ to $Na^+$ is crucial to achieving the intended effect of minimizing the formation of free iron. It's however well known to those in the art that a formulation cannot be made on the basis of the addition of any amount or ratio of ions.

Therefore any ratio of calcium and sodium ions pursuant to the present invention must be read as a suitable molar ratio of the respective salts comprising these ions. An easy way of obtaining the desired molar ratios of the ions is using their respective halide salts (e.g. chloride salts), as both $CaCl_2$ and $NaCl$ each comprise one ion of calcium and sodium, respectively. This results in the molar ratio of these salts being identical to molar ratio of the respective ions. In case salts are used that have more than one calcium and/or sodium ion, appropriate correction of the molar ratio is required when trying to compute that molar ratio of $Ca^{2+}$ to $Na^+$ from the respective molar ratio of the salts used.

Another advantage of the above two chloride salts of the calcium and sodium ions is that both are readily soluble in many solvents, particularly in water.

The above triazinones are well known per se as active ingredients against coccidial infections. In the context of the present invention, Toltrazuril is preferred.

The dosage rate of the triazinone may vary according to the animal species. Conventional dosage rates are 1 to 60 mg active ingredient per kg bodyweight (mg/kg) of the animal to be treated per day, preferably 5 to 40 mg/kg and especially preferably 10 to 30 mg/kg.

In the present context, polynuclear iron(III) polysaccharide complex compounds are understood as meaning complexes of the iron(III) ion with hydroxide ions ($OH^-$), aquo groups ($H_2O$) and oxygen (O) which are present in oligomeric or polymeric form and which are associated in their coordination sphere as complexes with one or more than one of the above oligomeric and polymeric carbohydrate compounds. This is why the compounds are also referred to as iron(III) hydroxide polysaccharide or iron(III) oxyhydroxy polysaccharide, where polysaccharide represents the corresponding oligomeric and polymeric carbohydrate compounds or their derivatives.

Polynuclear iron(III) complexes of this type are described for example in (D.S. Kudasheva et al., "Structure of Carbohydrate-bound Polynuclear Oxyhydroxide Nanoparticles in Parenteral Formulation", J. Inorg. Biochem. 98 (2004) 1757-1769; I. Erni et al "Chemical Characterization of Iron(III) Hydroxide-Dextrin Complexes" Arzneim.-Forsch./Drug Res. 34 (II) (1984) 1555-1559; F. Funk et al., "Physical and Chemical Characterization of Therapeutic Iron Containing Materials", Hyperfine Interactions 136 (2001) 73-95; E. London "The Molecular Formula and Proposed Structure of the Iron-Dextran Complex, IMFERON", J. Pharm. Sci. 93 (2004) 1838-1846; A. John "Neue Möglichkeiten der Eisenversorgung neugeborener Ferkel unter Beachtung biochemischer Aspekte" [Novel possibilities of supplying iron to new born piglets, taking account of biochemical aspects], Trächtigkeit und Geburt beim Schwein [Pregnancy and birth in pigs]: 8th Bernburger Biotechnology Workshop, Bernburg (2002) 89-94).

Since in many cases the precise composition of these compounds is not described in quantitative terms, and may also vary within the compounds, depending on the type of preparation, these polynuclear iron(III) polysaccharide compounds are understood as meaning all compounds which the skilled worker ascribes to this class of compounds.

Examples of polynuclear iron(III) polysaccharide complex compounds may be mentioned are: polynuclear iron(III) polysaccharide complex compounds in which a polynuclear β-FeO(OH) nuclear complex contains polymeric carbohydrate compounds associated at the free coordination sites, for example iron(III) dextran glucoheptonate (Gleptoferron), iron(III) dextran, iron(III) hydroxy polymaltose (iron(III) dextrin), nonstoichiometric compounds of β-FeO(OH) with saccharides and oligosaccharides "iron(III) sucrose" "iron(III) 'sugar'".

The formulation used according to the invention can be a solution, emulsion or suspension.

Solutions are prepared by dissolving the active ingredient, or active ingredients, in suitable solvents or solvent mixtures. If appropriate, further adjuvants such as solubilizers, antioxidants, preservatives, thickeners, adhesives, pH regulators, UV stabilizers or colorants are added.

Solvents which may be mentioned are: physiologically acceptable solvents such as water, alcohols, such as, for example, monohydric alkanols (for example ethanol or n-butanol), polyhydric alcohols such as glycols (for example ethylene glycol, propylene glycol, tetraglycol/glycofurol), polyethylene glycols, polypropylene glycols, glycerol; aromatically substituted alcohols such as benzyl alcohol, phenylethanol, phenoxyethanol; esters such as ethyl acetate, butyl acetate, benzyl benzoate, ethyl oleate; ethers such as alkylene glycol alkyl ethers (for example dipropylene glycol monomethyl ether, diethylene glycol monobutyl ether); ketones such as acetone, methyl ethyl ketone; aromatic and/or aliphatic hydrocarbons, vegetable or synthetic oils; glycerol formal, solketal (2,2-dimethyl-4-hydroxymethyl-1,3-dioxolane), N-methylpyrrolidone, 2-pyrrolidone, N,N-dimethylacetamide, glycofurol, dimethylisosorbitol, lauroglycol, propylene carbonate, octyldodecanol, dimethylformamide, and mixtures of the above-mentioned solvents.

Water is preferred as a solvent and/or suspension medium.

Solubilizers which may be mentioned are agents which promote the dissolution of the active ingredient in the main solvent or which prevent its precipitation. Examples for agents that prevent precipitation are polyvinylpyrrolidones.

Antioxidants are sulphites or metabisulphites such as potassium metabisulphite or sodium metabisulphite, sodium disulphite or potassium disulphite, ascorbic acid, isoascorbic acid, ascorbyl palmitate, gallic acid esters, butylhydroxytoluene, butylhydroxyanisole or tocopherols.

Synergists of these antioxidants may be: amino acids (for example alanine, arginine, methionine, cysteine), citric acid, tartaric acid, edetic acid or their salts, phosphoric acid derivatives or polyalcohols (polyethylene glycol).

Preservatives may be: benzyl alcohol, benzalkonium chloride, trichlorobutanol, p-hydroxybenzoate, n-butanol, chlorocresol, cresol, phenol, benzoic acid, citric acid, tartaric acid or sorbic acid.

Thickeners may be: inorganic thickeners such as bentonites, colloidal silica, aluminium stearates, organic thickeners such as cellulose derivatives, for example Hydroxypropylmethylcellulose 4000, polyvinyl alcohols and their copolymers, xanthan, acrylates and methacrylates, carboxymethylcellulose and its salts.

Adhesives are, for example, cellulose derivatives, starch derivatives, polyacrylates, natural polymers such as alginates, gelatin.

Adhesives which also have thickening properties may likewise be employed as thickeners.

pH regulators are pharmaceutically customary acids or bases. The bases include alkali metal hydroxides or alkaline earth metal hydroxides (for example NaOH, KOH), basic salts such as, for example, ammonium chloride, basic amino acids such as, for example, arginine, choline, meglumine, ethanolamines or else buffers such as tris(hydroxymethyl)aminomethane, citric acid buffers or phosphate buffers. The acids include, for example, hydrochloric acid, acetic acid, tartaric acid, citric acid, lactic acid, succinic acid, adipic acid, methanesulphonic acid, octanoic acid, linolenic acid, gluconolactone, and acidic amino acids such as, for example, aspartic acid.

UV stabilizers are, for example, substances from the class of the benzophenones, or novantisolic acid.

Colorants are all colorants which are approved for use on humans or animals and which may be dissolved or suspended.

Suspensions are prepared by suspending the active ingredient, or active ingredients, in a carrier liquid, if appropriate, with addition of further auxiliaries such as wetters, colorants, absorption accelerators, thickeners, adhesives, preservatives, antioxidants, UV stabilizers or antifoams.

The formulations used according to the invention are preferably "water based".

This means that, as a rule, they contain from $\geq 10$ to 90% by weight, preferably from $\geq 20$ to $\leq 80\%$ by weight, especially preferably from $\geq 30$ to $\leq 50\%$ by weight of water.

For example, the formulations as mentioned above may comprise further water-miscible solvents. Further water-miscible solvents which may be mentioned by way of example are preferably polyhydric aliphatic alcohols such as ethylene glycol, diethylene glycol, triethylene glycol, propylene glycol and glycerol; among these, propylene glycol is especially preferred.

Such further water-miscible solvents are usually present in concentrations of from $\geq 1$ to $\leq 45\%$ by weight, preferably from $\geq 1$ to $\leq 20\%$ by weight, especially preferably from $\geq 5$ to $\leq 10\%$ by weight. The addition of such polyhydric aliphatic alcohols also has the advantage of lowering the freezing point of the formulation.

The formulations used according to the invention as stated above can furthermore contain preservatives, if appropriate in combination with what are known as synergists. The preservatives are usually present in concentrations of from $\geq 0.01$ to $\leq 5\%$ by weight and specifically of $\geq 0.05$ to $\leq 1\%$ by weight.

If required, antioxidants which may be employed in the used formulations mentioned are, preferably, BHA or BHT. To ensure a sufficient preservation, the preservatives may be employed singly or else in combination with what are known as synergists. Synergists such as citric acid, tartaric acid, ascorbic acid or the sodium salt of editic acid are usually present in concentrations of from $\geq 0.01$ to $\leq 1\%$ by weight, specifically of $\geq 0.05$ to $\leq 0.15\%$ by weight.

Suspensions are prepared by suspending the solid active ingredient, or solid active ingredients, in any solvent described before in the context of solutions, if appropriate, with addition of further auxiliaries such as surfactants, dispersants, colorants, absorption accelerators, thickeners, adhesives, preservatives, antioxidants, UV stabilizers or antifoams, tonicity adjusters, antifreeze agents. Agents to control the state of flocculation of the suspended particles are also often added.

Surfactants may be anionic, cationic, ampholytic and/or non-ionic surfactants.

Anionic surfactants may for example be sodium lauryl sulphate, fatty alcohol ether sulphates, the monoethanolamine salt of mono/dialkyl polyglycol ether orthophosphoric acid esters, or lignosulphonates or dioctylsulphosuccinates such as sodium docusate.

A typical cationic surfactant is for example cetyltrimethylammonium chloride.

Typically used ampholytic surfactants are for example disodium N-lauryl-β-imino-dipropionate or lecithin.

Commonly used non-ionic surfactants are for example polyoxyethylated castor oil, polyoxyethylated sorbitan monooleate, sorbitan monostearate, ethyl alcohol, glycerol monostearate, polyoxyethylene stearate, alkylphenol polyglycol ethers and poloxamers (Pluronic®).

In the context of the present invention, anionic surfactants and non-ionic surfactants are preferred. Amongst the anionic surfactants soduium docusate is preferred and amongst the non-ionic surfactants Polysorbates, Copolymer Polyoxyl 35 - hydrogenated castor oil and Pluronic F-68 are preferred.

Amongst the Polysorbates, Polysorbate 20 and Polysorbate 80 are preferred.

Dispersants are additives that facilitate the wetting and suspending of the solid particle in the continuous phase. Examples are polyvinylalcohol, polyvinlypyrrolidones, or polyvinylacetate-copolymers. Amongst the dispersants, polyvinylpyrrolidones (PVP) are preferred.

Flocculation control agents are often salts like mineral or organic salts of alkaline, earth alkaline or earth metals. The addition of salt controls the electrostatic interaction of the suspended particles. Nonionic polymers or charged polymers may also be added. Examples for charged polyelectrolytes are sodium carboxymethylcellulose and natural polysaccharides like Xanthan. Examples for nonionic polymers may be polyethyleneglycols or hydroyiproplymethylcellulose. Their use depends on the ability to adsorb to the surfaces of the suspended particles and to bridge those particles.

Suitable antifoams are preferably those which are based on silicone, for example dimethicone or simethicone.

The formulations according to the invention are preferably "water based" suspensions. This means that, as a rule, they contain from ≥ 10 to 90% by weight, preferably from ≥ 20 to ≤ 80% by weight, especially preferably from ≥ 30 to ≤ 60% by weight of water. Those "water-based" suspensions may however comprise further water-miscible solvents.

The amount of formulation to be applied per administration depends on how much triazinone and active iron are to be administered in each case. One aims at relatively small volumes which can be applied readily and which vary depending on the animal species; for sucking pigs, for example, one aims at application volumes of from ≥ 0.3 to ≤ 2 mL, preferably from ≥ 0.5 to ≤ 1.5 mL.

The formulations according to the invention as stated above can furthermore contain preservatives, if appropriate in combination with what are known as synergists. The preservatives are usually present in concentrations of from ≥ 0.01 to ≤ 5% by weight and specifically of ≥ 0.05 to ≤ 1% by weight.

If required, antioxidants which may be employed in the formulations mentioned are, preferably, BHA or BHT. To ensure a sufficient preservation, the preservatives may be employed singly or else in combination with what are known as synergists.

Synergists such as citric acid, tartaric acid, ascorbic acid or the sodium salt of editic acid are usually present in concentrations of from ≥ 0.01 to ≤ 1% by weight, specifically of ≥ 0.05 to ≤ 0.15% by weight.

If appropriate, the formulations according to the invention may contain customary antifoams in concentrations of from ≥ 0.01 to ≤ 1% by weight.

The formulations according to the invention are preferably prepared by initially introducing the solvent, preferably water or the liquid carrier vehicle, preferably the iron(III)polysaccharide colloidal solution, and pre-dissolving or dispersing therein if appropriate auxiliaries and/or additives such as, for example, co-solvents, preservatives, antioxidants and viscosity-regulating additives, or, if desired, secondary active pharmaceutical ingredients and beneficial agents, such as, for example, $Ca^{2+}$ compounds.

In the preferred method, a second step involves introducing, into this initial solution, the triazinone, optionally in the form of a ready-made dispersion concentrate, using a powerful homogenizer and homogenizing the mixture until the finely divided suspension is obtained.. In the last step, finally, the desired pH is adjusted by addition of suitable pH regulators. Individual or all auxiliaries and/or additives as well as all beneficial agents, may, if appropriate, also be added after the last homogenization step to obtain the final volume or required dose; this may be advisable for example in the case of certain thickeners, whose structure is destroyed by the homogenization process. In the case of the pharmaceutically active iron compound this may be also obtained by the addition of the iron compound in form of a powder at this stage.

The formulations according to the invention are suitable for the combined control of Coccidia and iron deficiencies, in particular in animals. Using the formulations, it is possible to administer, to the animals, the anticoccidial triazinones and the iron simultaneously in a simple manner. The formulations may be used in animal keeping and animal breeding in livestock, breeding animals, zoo animals, laboratory animals, experimental animals and pets. The spectrum of action of the triazinones is, in principle, well known. Coccidia which may be mentioned individually are:

Mastigophora (Flagellata) such as, for example, Trypanosomatidae, for example Trypanosoma brucei, T. gambiense, T. rhodesiense, T. congolense, T. cruzi, T. evansi, T. equinum, T. lewisi, T. percae, T. simiae, T. vivax, Leishmania brasiliensis, L. donovani, L. tropica, such as, for example, Trichomonadidae, for example Giardia lamblia, G. canis.

Sarcomastigophora (Rhizopoda) such as, for example, Entamoebidae, for example Entamoeba histolytica, Hartmanellidae, for example Acanthamoeba sp., Hartmanella sp.

Apicomplexa (Sporozoa) such as, for example, Eimeridae, for example Eimeria ascervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Neospora caninum, N. hugesi, Cystisospora spec., Cryptosporidium spec. such as, for example, Toxoplasmadidae, for example Toxoplasma gondii, such as, for example, Sarcocystidae, for example Sarcocystis bovicanis, S. bovihominis, S. neurona, S. ovicanis, S. ovifelis, S. spec., S. suihominis such as, for example, Leucozoidae, for example Leucozytozoon simondi, such as, for example, Plasmodiidae, for example Plasmodium berghei, P. falciparum, P. malariae, P. ovale, P. vivax, P. spec., such as, for example, Piroplasmea, for example Babesia argentina, B. bovis, B. canis, B. spec., Theileria parva, Theileria spec., such as, for example, Adeleina, for example Hepatozoon canis, H. spec.

Furthermore Myxospora and Microspora, for example Glugea spec., Nosema spec.

Furthermore Pneumocystis carinii, and also Ciliophora (Ciliata) such as, for example, Balantidium coli, Ichthiophthirius spec., Trichodina spec., Epistylis spec.

Those genera and species of protozoans which lead to subclinical or clinical infections in pigs must be very particularly emphasized, especially: Eimeria debliecki, E. suis, E. scabra, E. perminuta, E. spinosa, E. polita, E. porci, E. neodebliecki, Isospora suis, Cryptosporidium, Toxoplasma gondii, Sarcocystis miescheriana, S. suihominis, Babesia trautmanni, B. perroncitoi, Balantidium coli.

Livestock and breeding animals to which the formulation according to the invention may be administered include mammals such as, for example, cattle, horses, sheep, pigs, goats, camels, water buffalos, donkeys, rabbits, fallow deer, reindeer, fur-bearing animals such as, for example, mink, chinchilla, racoon, birds such as, for example, chickens, geese, turkeys, ducks, pigeons, ostriches, bird species which are kept as companion animals and as zoo animals. They furthermore include farmed fish and ornamental fish. In this context, pigs, cattle, sheep and dogs of all species, subspecies and breeds may be particularly emphasized.

Laboratory and experimental animals include mice, rats, guinea pigs, golden hamsters, dogs and cats. Pets include dogs and cats.

The use in pigs is especially preferred. The formulations according to the invention are preferably applied to young animals, in particular shortly after birth, preferably in sucking pigs - piglets.

Usually, the formulations according to the invention are only applied once. Especially preferred formulations according to the invention permit the treatment of piglets in such a way that a sufficient supply of the piglets with iron in the first four weeks of life can be achieved with a single administration of from $\geq 0.5$ mL to $\leq 1.5$ mL of the formulation mL depending on the piglet weight even on the third day after birth, where a hemoglobin value of at least $\geq 8$ g/100 mL blood, preferably of more than $\geq 9$ g/100 mL blood, may be considered the indicator for a sufficient supply. In addition, the triazinone portion is intended to successfully control coccidia.

The formulations according to the invention may contain further active ingredients or components - singly or in suitable combinations -, such as, for example, nutrients, which include, for example, vitamins, minerals, and phosphorus compounds which are suitable as metabolic and immune stimulants:

Vitamins such as, for example, vitamin E, vitamins from the B series such as, for example, vitamin B12, vitamin C.

Minerals, preferably calcium or magnesium salts, in particular for example calcium gluconate, calcium glucoheptanoate, calcium saccharate, or calcium glycerophosphate

Phosphorus compounds, in particular pharmacologically acceptable organic phosphonic acid derivatives which are suitable as metabolic stimulants and tonics. Preferred examples which may be mentioned are the compounds toldimfos and, in particular, butaphosphane, which have already been known for a long time.

In another embodiment of the formulation the source of $Ca^{2+}$ and $Na^+$ to build the respective molar ratio thereof is $CaCl_2$ and NaCl. Those formulations usually have a total content of $CaCl_2$ and NaCl of $\geq 0.1\%$ (m/V) to $\leq 3\%$ (m/V). Preferred is $\geq 0.5\%$ (m/V) to $\leq 2\%$ (m/V), more preferred $\geq 0.5\%$ (m/V) to $\leq 1\%$ (m/V).

In another embodiment the formulation has a content of free iron of $\leq 3500$ ppm, preferably $\leq 2000$ ppm and more preferred $\leq 1000$ ppm.

Quantitative determination of free iron may be achieved dialysis of the sample with subsequent determination of dialized free iron with atomic absorption spectroscopy (AAS). Other quantitative methods are polarography and photometry.

The measured content of iron ions mainly consists of two sources of iron ions. It's apparent that any iron ions that are already dissolved in the solvent of the formulation are part of the free iron according to the present invention.

A second source of free iron is any iron that is present in the as free iron in form of small complex fragments, known in literature as labile iron. In other words such free iron is no longer stably bound in the polysaccharide complex either intrinsically or by the fact that the polysaccharide complex compound has partially degraded or otherwise become defective.

Those complex fragments are transferred into $Fe^{2+}$ and/or $Fe^{3+}$ ions by suitable sample preparation methods and measured as described above together with the $Fe^{2+}$ and $Fe^{3+}$ ions which are originally present in the formulations.

Hence, it is understood in the scope of this invention, that the content of iron ions determined by AAS, polarography or photometry refers to the content of $Fe^{2+}$ and/or $Fe^{3+}$ ions freely dissolved in the formulation in addition to those transferred into freely dissolved ions upon sample preparation, while the $Fe^{2+}$ ions are not directly measured, as they are transferred to $Fe^{3+}$ anyway during sample preparation or by any other active means to result in measureable free iron in the form of freely dissolved $Fe^{3+}$ ions.

The term "free" iron in the scope of this invention and measured by the above mentioned methods thereby comprises all iron compounds which may cause toxicity after administration by overloading the iron transport capacity of the iron transport proteins mentioned earlier.

Consequentially, any amount of free iron measured by one of the methods described just above is always identical or bigger than the actual amount of "free" iron according to the functional definition given just above, as all measurement methods measure the overall amount of freely dissolved iron and non-functional complexes thereof, while only a portion thereof maybe actually harmful to the subject getting it administered.

The above described "free" iron needs to be separated from "active iron". For the purpose of the present invention, "active iron" is the amount of iron stably present in the polynuclear iron(III) polysaccharide complex compounds. From that alone and in the context of the present invention, active iron cannot be $Fe^{2+}$, but must be $Fe^{3+}$.

Furthermore, if - in the context of the present invention - weight percentages or ratios are disclosed with regard to said active iron, such amounts must always be considered to be stated without the respective amounts/weights of complex formation agents (i.e. the polysaccharide complex).

In another embodiment the formulation, after storage at a temperature of $\geq$ 20 °C to $\leq$ 40 °C for 6 months, has a content of dissolved Fe(II) and/or Fe(III) ions of $\leq$ 3500 ppm, preferably $\leq$ 2000 ppm and more preferred $\leq$ 1000 ppm.

It is more preferred that the formulation has these contents after storage of 12 months, even more preferred after 24 months and most preferred after 36 months.

For example, the formulation, after storage at a temperature of $\geq$ 20 °C to $\leq$ 40 °C for 36 months, may have a content of free iron of $\leq$ 3500 ppm.

In another embodiment the formulation contains $\geq$ 0.1% (m/V) to $\leq$ 30% (m/V) of a triazinone, preferably Toltrazuril. This corresponds to $\geq$ 1 to $\leq$ 300 mg/mL. Preferred are $\geq$ 0.9 to $\leq$ 25% (m/V), corresponding to $\geq$ 9 to $\leq$ 250 mg/mL, especially preferably $\geq$ 1.8 to $\leq$ 15% (m/V), corresponding to $\geq$ 18 to $\leq$ 150 mg/mL, in particular $\geq$ 1.82 to $\leq$ 5% (m/V), corresponding to $\geq$ 18.2 to $\leq$ 50 mg of the triazinone, preferably toltrazuril in 1 mL.

As the result of the poor solubility of the triazinones, the latter are frequently present, in the formulations according to the invention, in finely suspended form.

Here, the dispersed triazinone has a particle size (measured by laser diffraction, Malvern Mastersizer® 3000) of d(v,90) < 30 $\mu$m, preferably d(v,90) < 20 $\mu$m, especially preferably d(v,90) < 10 $\mu$m, and very especially preferably d(v,90) = 7 $\mu$m or less. Also especially preferred is a particle size of d(v,95) < 10 $\mu$m.

For the purposes of the present invention, d(v,95) is to be understood as meaning a volume-related particle size distribution where 95% of all particles have a dimension (diameter) of this value or less. Usually, this information is referred to as d(95), but the more precise term d(v,95) may be chosen in order to make clear that it is a volume-related particle size distribution.

The names d(v,90), d(v,10) and the like are to be understood correspondingly. The particle sizes indicated here were determined with the laser diffraction method using the Mastersizer 3000 apparatus (dispersing unit LV Hydro) from Malvern and using the Fraunhofer diffraction evaluation mode since the refractive indices of the active ingredient particles are not known. Here, a suitable amount of the sample solution (e.g. 1mL) is predispersed, with stirring, with 4mL of a dispersion medium (0.02% aqueous dioctyl sodium sulphosuccinate solution). The dispersing unit LV Hydro is filled with the dispersion medium. Then, with stirring at 1750rpm, the sample dispersion is added dropwise to the dispersing medium into the dispersing unit LV Hydro until an obscuration ratio of 5 to 6% is reached., Stirring at 1750rpm and recirculation continues for two minutes. Then, under constant stirring, 6 min of ultrasound are applied and, after 2 min waiting period, the sample is measured.

In another embodiment the formulation contains $\geq$ 10% (m/V) to $\leq$ 30% (m/V) of active iron in form of the polynuclear iron(III) polysaccharide complex compound.

The terms X% (m/V) of active iron in this context refers to the volume related equivalent concentration of active iron as outlined above and implies a respective higher amount of polynuclear iron(III)polysaccharide complex compound. 10% (m/V) to $\leq$ 30% (m/V) then corresponds to 100 to 300 mg active iron in 1 mL.

Preferred are $\geq$ 17% (m/V) to $\leq$ 25% m/V, corresponding to $\geq$ 170 mg to $\leq$ 250 mg active iron in 1 mL, especially

preferably from $\geq$ 18% m/V to $\leq$ 23% m/V, corresponding to $\geq$ 180 mg to $\leq$ 230 mg active iron in 1 mL of the formulation.

Active iron refers to the percentage of iron which is present in the formulation in the form of the polynuclear iron(III) polysaccharide complex compound, as explained above.

The iron compounds in the formulations may be applied in concentrations of from 100 mg of active iron to 200 mg of active iron per unit dose as a single or multiple administration.

In another embodiment of the formulation the triazinone is toltrazuril and the polynuclear iron(III) polysaccharide complex compound is iron(III) dextran glucoheptonate. In a preferred embodiment the formulation is a parenteral, preferably injection formulation.

In another embodiment the formulations described herein is for use in the simultaneous treatment of coccidial infections and iron deficiencies.

Preferably the formulation is for use in the simultaneous treatment of coccidial infections and iron deficiencies in piglets in the period from birth to 10 days (preferably 5 days, more preferred 3 days) after birth.

In another embodiment, the formulation may consist of mixtures of polynuclear iron(III) polysaccharide complex solutions and $Ca^{2+}$ salts and/or $Na^{+}$-salts and either alone or in combination with a suitable number of other active pharmaceutical ingredients and/or, in general, agents beneficial to the treated animal.

It is understood that those typical formulations may also contain a preservative. It is convenient to preserve iron dextran solutions for injection, or solutions of iron(III) polysaccharide complexes in general, with up to 0.5% phenol, according to USP NF 29, p 1178.

Another aspect of the present invention is the use of the formulation according to the invention for the preparation of pharmaceuticals for the simultaneous treatment of coccidial infections and iron deficiencies.

Preferably the use is for the preparation of pharmaceuticals for the simultaneous treatment of coccidial infections and iron deficiencies in piglets in the period from birth to 10 days (preferably 5 days, more preferred 3 days) after birth.

It is also preferred that the use is for the preparation of parenteral, preferably injectable pharmaceuticals for the simultaneous treatment of coccidial infections and iron deficiencies in piglets in the period from birth to 10 days (preferably 5 days, more preferred 3 days) after birth.

The invention is further concerned with the use of a molar ratio of $Ca^{2+}$ to $Na^{+}$ from $\geq$ 0 to $\leq$ 3 for imparting a content of free iron of $\leq$ 3500 ppm (preferably $\leq$ 2000 ppm and more preferred $\leq$ 1000 ppm) in a formulation containing a triazinone selected from the list consisting of toltrazuril, ponazuril and diclazuril or a physiologically acceptable salt thereof and a polynuclear iron(III) polysaccharide complex compound, after storage at a temperature of $\geq$ 20 °C to $\leq$ 40 °C for 6 (preferably 12, more preferred 24, even more preferred 36) months. Regarding further details of the formulation reference is made to the preceding description in the interest of brevity.

The present invention will be further described with reference to the following examples without wishing to be limited by them. In the experiments, the effects of $CaCl_2$/NaCl on the concentration of free iron ions were studied.

**Examples:**

Analytical methods applied

In general, many methods are known to those skilled in the art to determine the amount of free iron in the formulations according to the present invention and the present invention is not bound to or depending on any particular method. More specifically, it's shown in the subsequent set of experiments, that a formulation comprising a certain ratio of $CaCl_2$:NaCl is beneficial in terms of stabilizing the complexed iron therein and that this is independent from the analytical methods applied to determine the free iron content in any of the formulations.

It is also understood that the content of free iron measured in absolute numbers may differ from method to method, as the methods vary in their ability to detect different species of labile iron (i.e. iron that is not yet freely dissolved but still partially complexed in a defective polysaccharide complex compound). The effect of minimizing the content of free iron according to the present invention, is however not relying on these differences, as any of the methods would - regardless of absolute value measured - always measure a decrease of free iron as a function of the molar ratio of $Ca^{2+}$ to $Na^{+}$, which is the relevant finding of this invention.

Polarography:

In case polarography is used to detect the amount of free iron in the formulations, the below described method is applied throughout the Examples of this invention.

A "Metrohm VA 797 Computrace" with mercury multi-mode electrode (MME), platinum auxiliary electrode and reference electrode "Ag/AgCl/KCl (3 mol/l)" was used as a polarograph.

The polarograph was equipped with an automatic dosing unit (Dosino 800) and a sampler (863 Compact VA Autosampler) The voltammetric process is performed in DPP mode using a dropping mercury electrode. Freely available iron ions are detected electrochemically on the mercury with Triethanolamine in an alkaline medium. A quantitative evaluation is performed using the standard addition process.

Any samples were prepared by transferring approximately 1 mL of a freshly shaken sample to a 10 mL volumetric flask on an analytical balance and the actual weight of the sample is recorded. The flask is filled to the calibrating mark with ultrapure water and shaken vigorously.

To produce a calibration solution and amount of $FeCl_3$, corresponding to approximately 100 mg of iron (accurate to 0.1 mg) is measured into a 100 mL volumetric flask, dissolved with ultrapure water, and approximately 1 mL of concentrated hydrochloric acid is added and filled to the calibrating mark with ultrapure water. To result in a nominal concentration of 1 g/L

To produce the supporting electrolyte, 4.48 g triethanolamine and 0.56 g potassium hydroxide is dissolved in 100 mL ultrapure water.

The actual polarography was thereafter conducted after having added 10 mL of the supporting electrolyte to the sample and then starting the hereinafter measurement protocol.

At first, a blank value measurement is taken. Thereafter, 1 mL sample solution is added. The measurement signal of the sample solution had to be within the verified linear range of the equipment. If the first readout was beyond the verified linear range, the sample quantity was reduced (e.g. from 1 mL to 100 $\mu$L) until the readout was well within the verified linear range.

Thereafter 100 $\mu$L of calibration solution was added twice. On the basis of the current (nA) readout of sample and current (nA) readout of the calibration solution (taken twice) the free iron content is computed from the peak heights using a linear regression mode, using the below equation:

$$W(Fe) = \frac{m(s)I(p)}{m(p)I(s)}[\frac{\mu g}{g}]$$

With W(Fe) being the content of free iron in a given sample [mg/kg], m(s) being the mass of iron [$\mu$g] in the calibration solution, I(p) being the current (nA) measured for the sample, I(s) being the current (nA) measured for the calibration solution and m(p) being the mass (g) of the sample measured.

*Dialysis*

In case dialysis is used to detect the amount of free iron in the formulations, the below described method is applied throughout the Examples of this invention. Dialysis - actually being rather purification than a measurement method - is hereinafter taken synonymously for the two step application of dialysis and subsequent inductively coupled plasma atomic optical emission spectrometry (ICP-OES) of the dialyzed samples.

The sample formulation is therefore placed in a dialysis tube to have the free iron diffusing into the water acceptor phase around the dialysis tube to thereafter quantitatively analyze the free iron in the acceptor phase by ICP-OES using a Varian Vista Pro device.

In any of the measurements taken hereinafter, a 6.3mm (8/32 inch) dialysis tube with 12000-14000 Dalton pore size was used (e.g. Medicell Int. Ltd. Dialysis tubing Visking Code DTV12000.01.000; or Roth Dialysis tube Visking, Cellulose type 8/32 inch, thickness 0.05mm, width 10mm). From said dialysis tube about 30cm are taken and closed with a knot on one end, which then is widened by pressurized air.

A bottle containing a representative sample of the formulation to be analyzed is shaken carefully to potentially re-suspend and homogenize and using a syringe about 3mL of the sample are withdrawn therefrom and filled into the dialysis tube. The tube is carefully placed into a 30mL test tube which is filled with 20mL of deionized water, while the open end of the dialysis tube is fixed at the test tube with a screw cap. The time allowed for dialysis is about 24h at a temperature of about 22°C.

Thereafter the dialysis tube is carefully removed and an aliquot of the acceptor phase water is taken and made subject to ICP-OES.

The aliquot volume varied depending on the content of free iron and optimum detection conditions of the ICP-OES device (i.e. either the aliquot amount is reduced or increased, if the free iron content is not well within the reliable range of measurement of the device). Accordingly, further dilution of the sample was sometimes necessary.

*Photometry*

In case photometry is used to detect the amount of free iron in the formulations, the below described method is applied throughout the Examples of this invention.

In general this analytical method is described well in Ph. Eur.2.2.25, clarifying that it relies on a color reaction of free iron with Bathophenantroline and subsequent spectrophotometric detection To generate a calibration standard, eight volumes of the iron standard solution (10 μg/mL $Fe^{3+}$ as $Fe(NO_3)_3$ in equal steps from 1 to 8mL) were each added up to 10mL with purified deionized water. To these solutions, 5mL hydroxylamin hydrochlorid solution (10%M/V), 10.0mL bathophenantroline solution (0.0332%M/V) and 5.0mL sodium acetate solution (10%M/V) were added and stored for later use.

0.8 to 1.0g of either sample formulation or calibration solution were each added into a 250mL flask and diluted with purified deionized water ad 250mL. From those an aliquot of 10mL is transferred into a separate flask. In case of a sample, again 5mL hydroxylamin hydrochlorid solution, 10.0mL bathophenantroline solution and 5.0mL sodium acetate solution were added (just as described above in the context of the calibration standard) and the solutions was mixed.

The mixture (with either the sample or the calibration solution) was stored for 30min at room temperature.

Next, the mixture is extracted twice with trichloromethane in a separatory funnel, while the two resulting trichloromethane phases are thereafter combined into a 50mL calibration flask and the volume completed ad 50mL with 2-propanol.

The solution is filled into a cuvette and the adsorption is measured against 2-propanol as reference standard at 533nm with a calibrated standard spectrophotometer of any type.

Method for preparing the formulations of the subsequent examples

Any formulations described and exemplified in the context of the present invention may be prepared with any method which is appropriate for a person skilled in the art.

Still, the formulations according to the subsequent examples were prepared according to the general process hereinafter, outlined by means of one specific example of a formulation having 2.72% M/V toltrazuril, 18.2% M/V active iron, 0.5% M/V phenol and any desired amount of any surfactants.

A 15%M/M pre-suspension of Toltrazuril in the iron (III) dextran glucoheptonate (Gleptoferron) colloidal solution was produced first. In a second step, the suspended toltrazuril in the crude suspension concentrate was micronized by wet bead milling. In a third step, the obtained suspension concentrate was diluted with calculated amounts of iron (III) dextran glucoheptonate (Gleptoferron) colloidal solution, surfactant(s) and preservative (phenol) stock solutions, and water to add up to the desired final concentration. The salts were also added during this third step.

The respective surfactants and preservatives were always added by means of adding a higher concentrated stock solution of the respective preservative and/or surfactants, while - in the context of the subsequent examples - the stock solutions were a 50% M/M Tween 20 stock solution, a 25% M/M Tween 80 stock solution, 5% M/M Docusate Sodium stock solution for the surfactants and a 5% M/M Phenol stock solution for the preservative.

The above referred to 15% M/M toltrazuril pre-suspension is prepared by filling a suitable amount of iron (III) dextran glucoheptonate (Gleptoferron) colloidal solution into a beaker and adding necessary amounts of surfactant to obtain 0.6% M/M Tween 20, 0.3% M/M Tween 80 and 0.15% M/M Docusate sodium. In this first step, Tween 80 and Tween 20 are added as 100% substance.

The solution is stirred with a dissolver disc. Then, under continuous stirring, the calculated amount of Toltrazuril is added to obtain a 15% M/M dispersion. The dispersion is stirred with the dissolver disc for 10 minutes and subsequently homogenized with an ultra-turrax at 9500rpm for another 10 minutes to produce a homogenous pre-suspension.

In the above referred to second step of micronization by wet bead milling, the milling chamber of the bead mill (Dyno-Mill KDL, Willy A Bachhofen AG, 300mL milling chamber volume) is filled with ZrO2/Y-millig beads (0.6-0.8mm diameter; 75% chamber fill grade). The pre-suspension is stirred with a paddle-stirrer and then conveyed through the bead mill by the use of a peristaltic pump at a flow rate of 30-40g/min. The milled suspension is discharged in a second beaker. The milling process is usually repeated two to three times to obtain the desired fine particle size of the toltrazuril.

The thereby obtained suspension concentrate is taken as stock-suspension and starting point of the third step mentioned above. For a 1000mL final suspension, 181.3g of the concentrate are weighed into a 2000mL beaker. The amounts of iron (III) dextran glucoheptonate, phenol stock solution, surfactant stock solutions and q.s. water to be added are calculated to obtain a final suspension containing 2.72% M/V toltrazuril, 18.2% M/V active iron, 0.5% M/V phenol and the desired surfactant concentrations.

The amounts of salt and surfactant solutions are calculated to obtain the %M/V percentages given in the respective examples. Under continuous stirring with a paddle stirrer, the suspension concentrate becomes diluted with the calculated amount of the iron (III) dextran glucoheptonate (Gleptoferron) colloidal solution. Next, the calculated amounts of NaCl and $CaCl_2$ are added and dissolved. Then the calculated amounts of the surfactant stock solutions and the phenol stock solution are added with a pipette. The suspension is then quantitatively transferred into a 1000mL volumetric flask and filled up to 1000mL with deionized water.

**Example 1:**

Formulations containing 3.64% M/V toltrazuril, 18.2% M/V active iron (added in the form of Gleptoferron 20% M/V iron), 0.5% M/V phenol, 0.09% M/V nonionic surfactant polysorbate 80 (Ph.Eur. name; also termed Tween™ 80 as trade name or polyoxiethylene sorbitan monostearate as the chemical name), 0.14% M/V nonionic surfactant polysorbate 20 (Ph. Eur.name; also termed Tween™ 20 as trade name or polyoxiethylene sorbitan monolaurate as the chemical name), and mixtures of sodium chloride and calcium chloride according to Table 1 were prepared.

The samples were analyzed by polarography for the content of free iron after the periods and at conditions of storage as stated in Table 1.

**Table 1: Formulations of Example 1 and free iron content measured as a function of salt ratio**

| | Salt or ratio | [% M/V] or ratio | Iron (10 d at 25°C) [g/100mL] | Iron (3 months at 30°C) [g/100mL] | Iron (3 months at 40°C) [g/100mL] |
|---|---|---|---|---|---|
| a | $CaCl_2$ | 0.3 | 0.0465 | 0.065 | 0.117 |
| | NaCl | 0.9 | | | |
| | $n(CaCl_2/NaCl)$ | 0.175 | | | |
| b | $CaCl_2$ | 1 | | | |
| | NaCl | 3 | | | |
| | $n(CaCl_2/NaCl)$ | 0.175 | | | |
| c | $CaCl_2$ | 0.5 | 0.044 | 0.059 | 0.108 |
| | NaCl | 2 | | | |
| | $n(CaCl_2/NaCl)$ | 0.132 | | | |

From the above data, it can be seen that the content of free iron is less at a lower molar ratio of $CaCl_2$:NaCl. The effect is more pronounced if more harsh (higher temperature) storage conditions.

For the formulations a and b of example 1, both having the identical molar ratio of $CaCl_2$:NaCl the respective average is displayed. The measured amount of free iron after 10 days did not deviate significantly. For example the deviation between a and b after 10 days at 25°C was 0.001 g/100mL.


**Example 2**

To investigate if the effect of molar ratio of $CaCl_2$:NaCl is irrespective of the respective amounts of surfactant and whether the particle size of toltrazuril in the formulation has an effect on the stabilization of iron complexes by the molar ratio of $CaCl_2$:NaCl, a comprehensive series of different formulations containing different ratios of $CaCl_2$:NaCl and particle sizes of toltrazuril [D(v, 95)] has been undertaken

All of the formulations of this experimental series contain 18.2% M/V of iron (as iron (III) dextran glucoheptonate (Gleptoferron, CAS No. 57680-55-4) colloidal solution); 2.72% M/V of suspended toltrazuril and 0.5% M/V of phenol as preservative. Respective details of the toltrazuril particle sizes and individual concentrations of salts and surfactants are specified in Table 2.

In this example, the method for detection of the free iron was dialysis.

Plotting the iron content measured over the ratio of $CaCl_2$:NaCl for each storage time and measurement method, and applying a linear fit to it, consistently shows a lowered free iron content at lower ratio of $CaCl_2$:NaCl, translating into a prove of the individual protective effect of a suitable ratio of $CaCl_2$:NaCl to the complexed iron pursuant to the present invention.

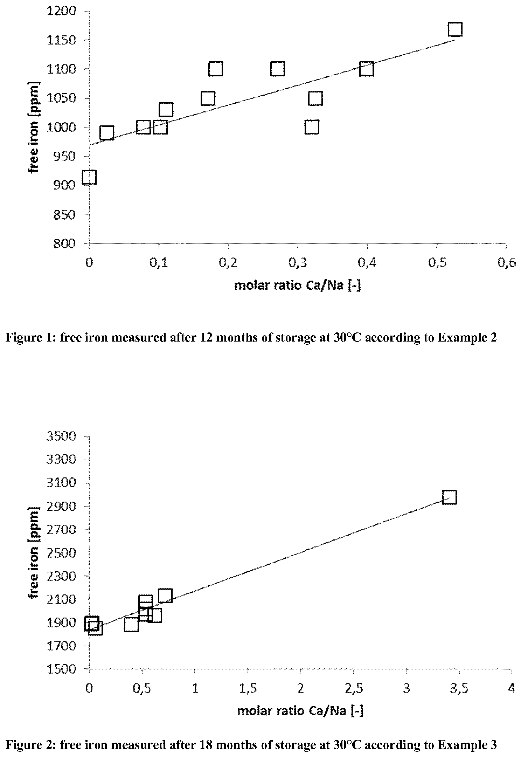

An exemplary graphical representation is given in Figure 1 for the free iron content pursuant to Table 3 plotted over the molar ratio of $CaCl_2$:NaCl after 12 months of storage (with multiple time ratios being represented by average free iron content of the multiple measurements and ending with ratios of $\leq 1$ to allow better graphical representation).

**Table 2: Formulations of Example 2**

| # | Tween 20 [% (M/V)] | Tween 80 [% (M/V)] | Docusate Sodium [% (M/V)] | NaCl [% (M/V)] | CaCl$_2$ [% (M/V)] | D(v,95) [μm] |
|---|---|---|---|---|---|---|
| 1 | 0.200 | 0.100 | 0.050 | 0.400 | 0.400 | 4.5 |
| 2 | 0.200 | 0.100 | 0.050 | 0.400 | 0.400 | 4.3 |
| 3 | 0.200 | 0.100 | 0.050 | 0.400 | 0.400 | 5.6 |
| 4 | 0.288 | 0.076 | 0.073 | 0.645 | 0.332 | 5.4 |
| 5 | 0.088 | 0.308 | 0.063 | 0.607 | 0.369 | 5.5 |
| 7 | 0.000 | 0.500 | 0.067 | 0.947 | 0.184 | 5.7 |
| 8 | 0.000 | 0.43 | 0.015 | 0.166 | 1.028 | 4.7 |
| 9 | 0.038 | 0.063 | 0.038 | 0.522 | 0.000 | 5.0 |
| 10 | 0.316 | 0.000 | 0.028 | 0.741 | 0.240 | 5.7 |
| 11 | 0.161 | 0.199 | 0.095 | 0.331 | 0.069 | 5.6 |
| 12 | 0.400 | 0.000 | 0.091 | 0.478 | 0.295 | 4.9 |
| 13 | 0.450 | 0.011 | 0.010 | 0.405 | 0.000 | 5.7 |
| 14 | 0.113 | 0.000 | 0.075 | 0.112 | 0.888 | 5.5 |
| 16 | 0.345 | 0.100 | 0.024 | 0.256 | 0.859 | 5.7 |
| 18 | 0.258 | 0.242 | 0.085 | 0.683 | 0.517 | 5.3 |
| 19 | 0.013 | 0.088 | 0.070 | 1.000 | 0.000 | 5.1 |
| 20 | 0.050 | 0.050 | 0.042 | 0.566 | 0.027 | 5.0 |
| 21 | 0.000 | 0.329 | 0.000 | 0.800 | 0.119 | 5.1 |
| 23 | 0.125 | 0.375 | 0.078 | 0.075 | 0.627 | 5.2 |
| 24 | 0.000 | 0.399 | 0.03 | 0.441 | 0.152 | 5.3 |
| 26 | 0.250 | 0.000 | 0.100 | 1.095 | 0.000 | 4.8 |
| 27 | 0.376 | 0.018 | 0.047 | 0.202 | 0.798 | 5.2 |
| 28 | 0.500 | 0.000 | 0.033 | 1.200 | 0.000 | 5.3 |
| 29 | 0.113 | 0.000 | 0.075 | 0.113 | 0.888 | 5.6 |
| 30 | 0.000 | 0.500 | 0.067 | 0.947 | 0.184 | 5.0 |

**Table 3: Measured free iron content of samples measured by dialysis after different times of storage at 30°C**

| No | n(CaCl$_2$/NaCl) | Iron [ppm] 2.5Mo | Iron [ppm] 6Mo | Iron [ppm] 12Mo |
|---|---|---|---|---|
| 1 | 0.526 | 400 | 840 | 1251 |
| 2 | 0.526 | 380 | 790 | 1165 |
| 3 | 0.526 | 420 | 780 | 1090 |
| 4 | 0.271 | 430 | 780 | 1100 |
| 5 | 0.320 | 430 | 780 | 1000 |
| 7 | 0.102 | 330 | 750 | 1000 |
| 8 | 3.260 | 380 | 900 | 1300 |
| 9 | 0.000 | 280 | 690 | 1100 |
| 10 | 0.170 | 280 | 730 | 1050 |

(continued)

| No | n(CaCl$_2$/NaCl) | Iron [ppm] 2.5Mo | Iron [ppm] 6Mo | Iron [ppm] 12Mo |
|----|------------------|------------------|----------------|-----------------|
| 11 | 0.109 | 290 | 620 | 1030 |
| 12 | 0.325 | 310 | 690 | 1050 |
| 13 | 0.000 | 290 | 710 | 910 |
| 14 | 4.153 | 350 | 820 | 1400 |
| 16 | 1.763 | 440 | 780 | 1100 |
| 18 | 0.398 | 370 | 680 | 1100 |
| 19 | 0.000 | 330 | 620 | 870 |
| 20 | 0.025 | 370 | 690 | 990 |
| 21 | 0.078 | 350 | 690 | 1000 |
| 23 | 4.376 | 340 | - | 1200 |
| 24 | 0.181 | 320 | - | 1100 |
| 26 | 0.000 | 290 | - | 810 |
| 27 | 2.076 | 350 | - | 1100 |
| 28 | 0.000 | 270 | - | 880 |
| 29 | 4.151 | 310 | - | 1100 |
| 30 | 0.102 | 240 | - | 1000 |

**Example 3**

To further investigate the influence of molar ratio of CaCl$_2$:NaCl in view of its dependency on the presence of an ionic surfactant a third series of formulations, mostly identical to Example 2, but not comprising docusate sodium was prepared and stored and analyzed accordingly.

**Table 4: Formulations of Example 3**

| No | Tween 20 [% (M/V)] | Tween 80 [% (M/V)] | NaCl [% (M/V)] | CaCl$_2$ [% (M/V)] | D(v,95) [μm] |
|----|--------------------|--------------------|----------------|--------------------|--------------|
| 1 | 0.200% | 0.100% | 0.350% | 0.350% | 4.8 |
| 2 | 0.200% | 0.100% | 0.350% | 0.350% | 5.1 |
| 3 | 0.200% | 0.100% | 0.350% | 0.350% | 4.4 |
| 4 | 0.317% | 0.128% | 0.816% | 0.142% | 4.8 |
| 5 | 0.077% | 0.265% | 0.009% | 0.409% | 5.4 |
| 6 | 0.388% | 0.005% | 0.084% | 0.792% | 4.8 |
| 7 | 0.171% | 0.249% | 0.965% | 0.032% | 4.6 |
| 8 | 0.332% | 0.024% | 0.540% | 0.459% | 4.5 |
| 9 | 0.219% | 0.045% | 0.772% | 0.012% | 4.3 |
| 10 | 0.251% | 0.195% | 0.044% | 0.723% | 4.5 |
| 11 | 0.422% | 0.004% | 0.431% | 0.381% | 4.3 |
| 12 | 0.113% | 0.084% | 0.928% | 0.071% | 4.4 |
| 13 | 0.449% | 0.001% | 0.855% | 0.104% | 4.5 |
| 14 | 0.186% | 0.003% | 0.000% | 1.000% | 4.5 |
| 15 | 0.093% | 0.188% | 0.520% | 0.121% | 4.3 |

(continued)

| No | Tween 20 [% (M/V)] | Tween 80 [% (M/V)] | NaCl [% (M/V)] | $CaCl_2$ [% (M/V)] | D(v,95) [μm] |
|----|--------------------|--------------------|---------------|--------------------|--------------|
| 16 | 0.235% | 0.015% | 0.581% | 0.335% | 4.4 |
| 17 | 0.155% | 0.000% | 0.352% | 0.270% | 4.2 |
| 18 | 0.096% | 0.055% | 0.324% | 0.324% | 3.8 |
| 19 | 0.089% | 0.069% | 0.296% | 0.653% | 4.0 |
| 20 | 0.000% | 0.214% | 0.133% | 0.864% | 4.1 |
| 21 | 0.440% | 0.010% | 0.891% | 0.054% | 4.6 |
| 22 | 0.064% | 0.228% | 0.404% | 0.087% | 4.1 |
| 23 | 0.291% | 0.119% | 0.217% | 0.185% | 4.8 |
| 24 | 0.050% | 0.137% | 0.638% | 0.356% | 3.9 |
| 25 | 0.210% | 0.034% | 0.064% | 0.581% | 4.6 |
| 26 | 0.132% | 0.282% | 0.458% | 0.541% | 4.2 |
| 27 | 0.342% | 0.108% | 0.193% | 0.292% | 4.3 |
| 28 | 0.178% | 0.163% | 0.169% | 0.231% | 4.1 |

Dialysis and polarography were used to detect free iron. Results after 6 months at 30°C are shown in Table 5. Results after 18 months at 30°C are shown in Table 6

**Table 5: Measured free iron content of samples measured by dialysis after 6 months at 30°C**

| No | $n(CaCl_2/NaCl)$ | Iron [ppm] |
|----|------------------|------------|
| 1 | 0.53 | 700 |
| 2 | 0.53 | 670 |
| 3 | 0.53 | 690 |
| 4 | 0.09 | 580 |
| 5 | 23.37 | 710 |
| 6 | 4.98 | 730 |
| 7 | 0.02 | 590 |
| 8 | 0.45 | 700 |
| 9 | 0.01 | 600 |
| 10 | 8.57 | 750 |
| 11 | 0.47 | 670 |
| 12 | 0.04 | 580 |
| 13 | 0.06 | 660 |
| 14 | n.a. | - |
| 15 | 0.12 | 690 |
| 16 | 0.30 | 700 |
| 17 | 0.40 | 670 |
| 18 | 0.53 | 650 |
| 19 | 1.16 | 700 |
| 20 | 3.41 | 720 |

(continued)

| No | n(CaCl$_2$/NaCl) | Iron [ppm] |
|---|---|---|
| 21 | 0.03 | 580 |
| 22 | 0.11 | 670 |
| 23 | 0.45 | 750 |
| 24 | 0.29 | 660 |
| 25 | 4.78 | 760 |
| 26 | 0.62 | 780 |
| 27 | 0.80 | 810 |
| 28 | 0.72 | 810 |

**Table 6: Measured free iron content of samples measured by polarography after 18 months at 30°C**

| No | n(CaCl$_2$/NaCl) | Iron [ppm]C |
|---|---|---|
| 1 | 0.53 | 2076 |
| 2 | 0.53 | 2018 |
| 3 | 0.53 | 1970 |
| 7 | 0.02 | 1886 |
| 13 | 0.06 | 1853 |
| 17 | 0.40 | 1880 |
| 20 | 3.41 | 2980 |
| 21 | 0.03 | 1897 |
| 26 | 0.62 | 1958 |
| 28 | 0.72 | 2131 |

The effect molar ratio of CaCl$_2$:NaCl is visualized in Figure 2 based on the data of Table 6, clarifying that if a low free iron content (e.g. below 2500 ppm) of the formulation is intended to be achieved even after prolonged storage, a corresponding low molar ratio of CaCl$_2$:NaCl must be ensured.

**Example 4:**

The foregoing examples demonstrated the effect of a low molar ratio of CaCl$_2$:NaCl against release of free iron ions or small complex fragments from the polynuclear iron(III) polysaccharide complex upon storage over time and at higher temperatures. Consequently, the protective effect should be at maximum if the ratio approaches 0 as it is the case when no or very small amounts of Ca$^{2+}$ ions are present, e.g. from impurities.

To verify on an otherwise identical formulation basis, specific suspensions of other than content of Ca$^{2+}$ and Na$^+$ ions (NaCl or CaCl$_2$) identical composition were prepared and checked for the respective stability of iron therein (see Table 7 & Table 9). Therefore suspensions containing only NaCl and only CaCl$_2$ were prepared, stored at 40°C for a couple of months and analyzed for the content of free iron in course of an Example 4a.

Within another arm of this setup and to verify that the effect is not restricted and/or related to any other agent in a formulation but truly relates to a ratio while both ions (Ca2+ and Na+) are present (see Table 8 & Table 10) solutions containing only NaCl and only CaCl$_2$ together with an iron (III) dextran glucoheptonate were prepared, stored at 40°C for a couple of months and analyzed for the content of free iron (Example 4b).

In both arms (4a and 4b) analysis was done via the dialysis method, as indicated below.

The full compositions of the respective formulations are outlined in Table 7 and Table 8, while the remainder to 100% [M/V] is water.

**Table 7: Formulations of Example 4a that contain no NaCl or CaCl2 in suspension**

| No | Toltrazuril % [M/V] | Iron (III) as Gleptoferron complex % [M/V] | Tween 80 % [M/V] | Tween 20 % [M/V] | Phenol % [M/V] | NaCl [% M/V)] | CaCl2 [% M/V)] |
|----|------|------|------|------|-----|------|------|
| 1 | 1.82 | 18.2 | 0.07 | 0.13 | 0.5 | 0.9 | 0 |
| 2 | 1.82 | 18.2 | 0.07 | 0.13 | 0.5 | 0 | 0.9 |
| 3 | 1.82 | 18.2 | 0.07 | 0.13 | 0.5 | 0.55 | 0 |
| 4 | 1.82 | 18.2 | 0.07 | 0.13 | 0.5 | 0.9 | 0 |
| 5 | 1.82 | 18.2 | 0.07 | 0.13 | 0.5 | 2.0 | 0 |
| 6 | 1.82 | 18.2 | 0.07 | 0.13 | 0.5 | 0 | 0.55 |
| 7 | 1.82 | 18.2 | 0.07 | 0.13 | 0.5 | 0 | 0.9 |
| 8 | 1.82 | 18.2 | 0.07 | 0.13 | 0.5 | 0 | 2.0 |

**Table 8: Formulations of Example 4b that contain no NaCl or CaCl2 and only Iron(III) complex in solution**

| No | Iron (III) as Gleptoferron complex % [M/V] | NaCl [% M/V)] | CaCl2 [% M/V)] |
|----|------|------|------|
| 9 | 18.2 | 0 | 0 |
| 10 | 18.2 | 0.55 | 0 |
| 11 | 18.2 | 0.9 | 0 |
| 12 | 18.2 | 2.0 | 0 |
| 13 | 18.2 | 0 | 0.54 |
| 14 | 18.2 | 0 | 0.86 |
| 15 | 18.2 | 0 | 2.0 |

**Table 9: Free iron content of respective samples measured by dialysis (storage at 40°C) taken from Example 4a**

| No | Iron [ppm] Start | Iron [ppm] 1 Month | Iron [ppm] 2 Month | Iron [ppm] 3 Months | Iron [ppm] 5 Months | Iron [ppm] 6 Months |
|----|------|------|------|------|------|------|
| 1 | 97 |  | 760 |  | 1300 |  |
| 2 | 130 |  | 960 |  | 1500 |  |
| 3 | 92 | 680 |  | 1000 |  | 1500 |
| 4 | 54 | 600 |  | 990 |  | 1400 |
| 5 | 44 | 540 |  | 890 |  | 1300 |
| 6 | 96 | 780 |  | 1100 |  | 1600 |
| 7 | 88 | 800 |  | 1200 |  | 1600 |
| 8 | 80 | 750 |  | 1200 |  | 1600 |

**Table 10: Free iron content of respective samples measured by dialysis (storage at 40°C) taken from Example 4b**

| No | Iron [ppm] Start | Iron [ppm] 1 Month | Iron [ppm] 3 Months | Iron [ppm] 6 Months |
|----|------|------|------|------|
| 9 | 110 | 720 | 1200 | 1500 |

(continued)

| No | Iron [ppm] Start | Iron [ppm] 1 Month | Iron [ppm] 3 Months | Iron [ppm] 6 Months |
|---|---|---|---|---|
| 10 | 72 | 650 | 1100 | 1300 |
| 11 | 64 | 570 | 930 | 1300 |
| 12 | 68 | 520 | 810 | 1200 |
| 13 | 95 | 710 | 1100 | 1500 |
| 14 | 94 | 680 | 1200 | 1600 |
| 15 | 67 | 670 | 1100 | 1600 |

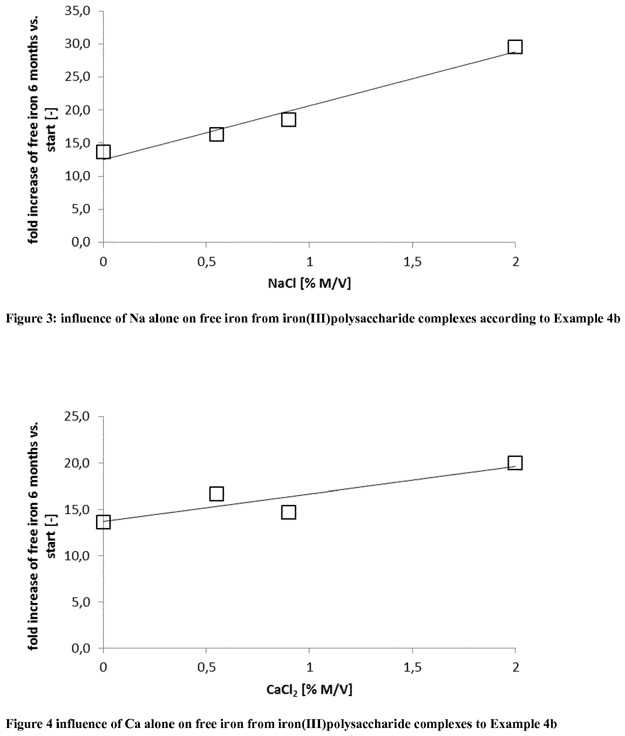

Taking the above data from Table 8 and Table 10 and plotting the increase of free iron (e.g. after 6 months) individually over sodium- and calcium chloride content, one can see that neither sodium nor calcium individually reduce the formation of free iron. Those data evidence, that the molar ratio of CaCl2:NaCl is decisive, as actually both negatively influence the free iron amounts with increasing concentration thereof (Figure 3& Figure 4).

**Claims**

1. A formulation comprising a triazinone selected from the list consisting of toltrazuril, ponazuril and diclazuril, a polynuclear iron(III)polysaccharide complex compound, and a molar ratio of $Ca^{2+}$ to $Na^+$ from $\geq 0$ to $\leq 3$, with the exception of sodium propionate and/or sodium benzoate being the sole source of $Na^+$ in the formulation when the molar ratio of $Ca^{2+}$ to $Na^+$ is 0.

2. A formulation according to claim 1, with the further exception of those formulations being selected from the list consisting of

   a. a formulation, consisting of 3.5% toltrazuril, 17.8% of iron (as Gleptoferron), 0.1% of sorbitan monooleate, 10% diethylene glycol monoethyl ether, 1% of sodium chloride, and Water ad 100%,
   b. a formulation, consisting of 3.5% toltrazuril, 19.3% of iron (as Gleptoferron), 0.3% of Propylene Glycol Monolaurate, 0.1% of copolymer Polyoxyl 35 - hydrogenated castor oil, 1% Sodium Chloride, and Water ad 100%,
   c. a formulation, consisting of 4.2% toltrazuril, 13.5% of iron (as Gleptoferron), 0.1% Sodium Docusate, 0.1% silicone emulsion, 1.5% sodium chloride, and Water ad 100%,
   d. a formulation, consisting of 4.2% toltrazuril, 13.5% of iron (as Gleptoferron), 0.1% Sodium Docusate, 5% diethylene glycol monoeethyl ether, 0.1% silicone emulsion, 1% sodium chloride, and Water ad 100%,
   e. a formulation, consisting of 4.2% toltrazuril, 13.5% of iron (as Gleptoferron), 0.6% Phenole, 0.2% Sodium Docusate, 0.1% simethicone emulsion USP 30%, 0.5% colloidal silicone dioxide, 1% povidone, 0.5% sodium chloride, and Water ad 100%,
   f. a formulation, consisting of 4.2% toltrazuril, 13.5% of iron (as Gleptoferron), 0.6% Phenole, 0.2% Sodium Docusate, 0.1% simethicone emulsion USP 30%, 0.5% colloidal silicone dioxide, 1% povidone, 1% sodium chloride, and Water ad 100%,
   g. a formulation, consisting of 4.2% toltrazuril, 13.5% of iron (as Gleptoferron), 0.6% Phenole, 0.1% Sodium Docusate, 0.1% simethicone emulsion USP 30%, 0.5% colloidal silicone dioxide, 1% povidone, 1% sodium chloride, and Water ad 100%,
   h. a formulation, consisting of 2.9% toltrazuril, 18.8% of iron (as Gleptoferron), 0.6% Phenole, 0.1% Sodium Docusate, 0.1% simethicone emulsion USP 30%, 0.5% colloidal silicone dioxide, 1% povidone, 1% sodium chloride, and Water ad 100%,
   i. a formulation, consisting of 2.9 % toltrazuril, 18.8 % of iron (as Gleptoferron), 0.3 % of sorbitan monooleate, 0.1 % of copolymer 15 Polyoxyl 35 - hydrogenated castor oil, 1.5% of sodium chloride and water,
   j. a formulation, consisting of 4.2 % toltrazuril, 13.5 % of iron (as Gleptoferron), 0.3 % of sorbitan monooleate, 0.1 % of copolymer Polyoxyl 35 - hydrogenated castor oil, 1.5% of sodium chloride and water, and
   k. a formulation, consisting of 4.2 % toltrazuril, 13.5 % of iron (as Gleptoferron), 1 % of sodium chloride, 1 % of polyvinylpyrrolidone, 0.6% of phenol, 0.5% of colloidal silicon dioxide, 0.1 % of sodium docusate, 0.1 % of simethicon emulsion and water.

3. The formulation according to claim 1 or 2, wherein the formulation has a molar ratio of $Ca^{z+}$ to $Na^+$ from $\geq 0$ to $\leq 3$.

4. The formulation according to claim 1, 2 or 3, wherein $Ca^{2+}$ and $Na^+$ are present as their respective chloride salts.

5. The formulation according to claim 4, wherein the total content of $CaCl_2$ and NaCl is from $\geq$ 0.1% (m/V) to $\leq$ 3% (m/V), preferably $\geq$ 0.5% (m/V) to $\leq$ 2% (m/V), more preferably $\geq$ 0.5% (m/V) to $\leq$ 1% (m/V).

6. The formulation according to any one of the previous claims, wherein active iron in the form of the polynuclear iron(III) polysaccharide complex compound is present from $\geq$ 10% (m/V) to $\leq$ 30% (m/V).

7. The formulation according to any one of the previous claims, wherein the triazinone is toltrazuril and the polynuclear iron(III) polysaccharide complex compound is iron(III)dextran glucoheptonate.

8. The formulation according to any one of the previous claims, wherein the formulation also comprises at least one non-ionic surfactant.

9. The formulation according to any one of the previous claims for use in the simultaneous treatment of coccidial infections and iron deficiencies.

10. The formulation for use according to claim 8 for use in parenteral application.

11. The formulation for use according to claim 9 for use in injection application.

12. The formulation for use according to any one of claims 8 to 10 for use in the simultaneous treatment of coccidial infections and iron deficiencies in piglets in the period from birth to 10 days after birth.

13. Use of $Ca^{z+}$ and $Na^+$ in a molar ratio of $Ca^{z+}$ to $Na^+$ from $\geq$ 0 to $\leq$ 3for imparting a content of dissolved free iron of $\leq$ 3500 ppm in a formulation comprising a triazinone selected from the list consisting of toltrazuril, ponazuril and diclazuril, and a polynuclear iron(III) polysaccharide complex compound, after storage at a temperature of $\geq$ 20 °C to $\leq$ 40 °C for at least 6 months.

**Figure 1: free iron measured after 12 months of storage at 30°C according to Example 2**

**Figure 2: free iron measured after 18 months of storage at 30°C according to Example 3**

Figure 3: influence of Na alone on free iron from iron(III)polysaccharide complexes according to Example 4b

Figure 4 influence of Ca alone on free iron from iron(III)polysaccharide complexes to Example 4b

Europäisches
Patentamt

European
Patent Office

Office européen
des brevets

# EUROPEAN SEARCH REPORT

Application Number

EP 18 17 6139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2014/086960 A1 (CEVA SANTE ANIMALE) 12 June 2014 (2014-06-12) | 1,2,6-13 | INV. A61K31/53 A61K45/06 A61K9/00 A61K31/555 A23K50/60 A23L33/10 A23L33/165 A61K47/60 |
| Y | * page 13, lines 6-18 * <br> * page 14, lines 13-25 * <br> * page 2, line 18 - page 3, line 2 * <br> * page 17, lines 1-24 * <br> * page 18, lines 1-8,13-24; examples 2-14 * <br> * page 19, lines 8-17 * <br> * pages 21-23; claims 1-7,15-16; examples forms 4,5 * <br> * page 15, lines 12-25,13-14; claim 6; examples Forms 6,7;9-14 * <br> * page 17, lines 8,23 - page 18, lines 7,15,18,20 * <br> * page 19, lines 16-17 * | 1,13 | |
| Y | WO 01/10465 A1 (BAYER AG) 15 February 2001 (2001-02-15) | 1,13 | |
| A | * page 4, lines 27-29 * <br> * page 5, lines 16-25 * <br> * page 6, lines 20-24 * <br> * page 9, lines 19-24 * <br> * page 3, line 29 - page 4, line 10; claims 4,5,11 * | 3-5 | TECHNICAL FIELDS SEARCHED (IPC) <br> A61K A23K A23L |
| Y | WO 2007/062546 A1 (CHONGQING PHARM RES INST CO) 7 June 2007 (2007-06-07) | 1,13 | |
| A | * paragraphs [0002], [0005], [0006], [0008] * <br> * paragraphs [0009], [0011], [0013], [0014], [0030] - [0034]; claims 1,3,16 * | 3-5 | |

-/--

The present search report has been drawn up for all claims

| Place of search | Date of completion of the search | Examiner |
|---|---|---|
| The Hague | 3 December 2018 | Kanbier, Titia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Europäisches
Patentamt
European
Patent Office
Office européen
des brevets

Application Number

EP 18 17 6139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/146155 A1 (CEVA SANTE ANIMALE) 23 December 2010 (2010-12-23) * paragraphs [0002], [0003], [0005], [0008], [0028], [0031] - [0032], [0038], [0042], [0044]; claims 6,9-15; examples 2-4 * * paragraphs [0008], [0012], [0017], [0023], [0030], [0036], [0042]; claims 1,3,5 * | 1,13 | |
| A | EP 0 091 762 A1 (ORION YHTYMAE OY) 19 October 1983 (1983-10-19) * page 1, lines 10-13,19-22 - page 2, lines 6-9; claims 1,3-5 * * page 2, lines 12-14,20-24 * * page 3, lines 9-12 * * page 4, lines 5-6 * * page 3, lines 4-6 - page 9, lines 8-23; examples 1,2,4,5 * | 1,13 | |
| A | US 3 076 798 A (ARTHUR MUELLER ET AL) 5 February 1963 (1963-02-05) * column 3, lines 23-33 * * column 4, line 10; example 2 * | 1,13 | TECHNICAL FIELDS SEARCHED (IPC) |
| A | US 4 994 283 A (MEHANSHO H ET AL) 19 February 1991 (1991-02-19) * column 1, lines 16-21,39-42 - column 2, lines 7-15 * * column 4, lines 34-42,53-57 * | 1,4,13 | |

The present search report has been drawn up for all claims

| Place of search | Date of completion of the search | Examiner |
|---|---|---|
| The Hague | 3 December 2018 | Kanbier, Titia |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 18 17 6139

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2018

| Patent document cited in search report | Publication date | Patent family member(s) | | Publication date |
|---|---|---|---|---|
| WO 2014086960 A1 | 12-06-2014 | AU | 2013353984 A1 | 11-06-2015 |
| | | BR | 112015013179 A2 | 11-07-2017 |
| | | CA | 2892888 A1 | 12-06-2014 |
| | | CL | 2015001565 A1 | 23-10-2015 |
| | | CN | 105007944 A | 28-10-2015 |
| | | EP | 2740492 A1 | 11-06-2014 |
| | | EP | 2928498 A1 | 14-10-2015 |
| | | JP | 6363092 B2 | 25-07-2018 |
| | | JP | 2016505558 A | 25-02-2016 |
| | | JP | 2018138569 A | 06-09-2018 |
| | | KR | 20150107734 A | 23-09-2015 |
| | | PH | 12015501219 A1 | 17-08-2015 |
| | | RU | 2015127020 A | 13-01-2017 |
| | | UA | 115460 C2 | 10-11-2017 |
| | | US | 2015313940 A1 | 05-11-2015 |
| | | WO | 2014086960 A1 | 12-06-2014 |
| WO 0110465 A1 | 15-02-2001 | AR | 025001 A1 | 06-11-2002 |
| | | AT | 270104 T | 15-07-2004 |
| | | AU | 771058 B2 | 11-03-2004 |
| | | BG | 65304 B1 | 31-01-2008 |
| | | BR | 0013010 A | 30-04-2002 |
| | | CA | 2378424 A1 | 15-02-2001 |
| | | CN | 1368891 A | 11-09-2002 |
| | | CO | 5190677 A1 | 29-08-2002 |
| | | CU | 23367 A3 | 14-04-2009 |
| | | CZ | 20020458 A3 | 12-06-2002 |
| | | DE | 19937116 A1 | 08-02-2001 |
| | | DE | 50006974 D1 | 05-08-2004 |
| | | DK | 1206281 T3 | 25-10-2004 |
| | | EE | 200200060 A | 15-04-2003 |
| | | EP | 1206281 A1 | 22-05-2002 |
| | | ES | 2223549 T3 | 01-03-2005 |
| | | GT | 200000123 A | 01-08-2000 |
| | | HK | 1049615 A1 | 10-11-2006 |
| | | HN | 2000000115 A | 02-02-2001 |
| | | HR | P20020198 A2 | 29-02-2004 |
| | | HU | 0203619 A2 | 28-02-2003 |
| | | IL | 147553 A | 05-09-2006 |
| | | JP | 5522877 B2 | 18-06-2014 |
| | | JP | 2003506416 A | 18-02-2003 |
| | | MA | 25427 A1 | 01-04-2002 |
| | | MX | PA02001254 A | 12-08-2002 |
| | | NO | 20020526 A | 01-02-2002 |
| | | NZ | 516969 A | 26-03-2004 |
| | | PE | 04002001 A1 | 11-06-2001 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 18 17 6139

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2018

| Patent document cited in search report | | | Publication date | Patent family member(s) | | | Publication date |
|---|---|---|---|---|---|---|---|
| | | | | PL | 353405 | A1 | 17-11-2003 |
| | | | | PT | 1206281 | E | 29-10-2004 |
| | | | | SI | 1206281 | T1 | 31-12-2004 |
| | | | | SK | 1682002 | A3 | 04-06-2002 |
| | | | | TW | I238719 | B | 01-09-2005 |
| | | | | UA | 72268 | C2 | 15-12-2004 |
| | | | | US | 6548079 | B1 | 15-04-2003 |
| | | | | UY | 26269 | A1 | 16-03-2001 |
| | | | | WO | 0110465 | A1 | 15-02-2001 |
| | | | | ZA | 200200215 | B | 25-09-2002 |
| WO 2007062546 | A1 | | 07-06-2007 | NONE | | | |
| WO 2010146155 | A1 | | 23-12-2010 | FR | 2946882 | A1 | 24-12-2010 |
| | | | | WO | 2010146155 | A1 | 23-12-2010 |
| EP 0091762 | A1 | | 19-10-1983 | AU | 553154 | B2 | 03-07-1986 |
| | | | | CS | 8302291 | A2 | 13-06-1985 |
| | | | | DD | 209611 | A5 | 16-05-1984 |
| | | | | DE | 3360380 | D1 | 14-08-1985 |
| | | | | DK | 143683 | A | 08-10-1983 |
| | | | | EP | 0091762 | A1 | 19-10-1983 |
| | | | | FI | 65913 | B | 30-04-1984 |
| | | | | HU | 188446 | B | 28-04-1986 |
| | | | | IE | 54475 | B1 | 25-10-1989 |
| | | | | PL | 241367 | A1 | 16-07-1984 |
| | | | | US | 4476114 | A | 09-10-1984 |
| | | | | ZA | 8302045 | B | 28-12-1983 |
| US 3076798 | A | | 05-02-1963 | NONE | | | |
| US 4994283 | A | | 19-02-1991 | NONE | | | |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 2718799 A
- DE 2413722 A
- WO 9962519 A
- EP 2740469 A1
- EP 2740470 A1
- WO 2008145281 A2

- WO 2014086960 A1
- US 20170202802 A1
- CN 102835553

**Non-patent literature cited in the description**

- National Research Council, Nutrient Requirements of Domestic Animals, No. 2. Nutrient Requirements of Swine. National Academy of Sciences, 1973
- Don't forget coccidiosis, update on Isosporosis in piglets. *Part I, Pig Progress,* vol. 17 (2), 12-14
- **MUNDT., H.-C. ; A. DAUGSCHIES ; V. LETKOVA.** piglet coccidiosis diagnostics. *Part II, Pig Progress,* 2001, vol. 17 (4), 18-20
- **MUNDT, H.-C. ; G.-P1 MARTINEAU ; K. LARSEN.** control of coccidiosis. *Part III, Pig Progress,* 2001, vol. 17 (6), 18-19
- *CHEMICAL ABSTRACTS,* 101831-37-2
- *CHEMICAL ABSTRACTS,* 69004-03-1
- *CHEMICAL ABSTRACTS,* 9004-66-4
- *CHEMICAL ABSTRACTS,* 57680-55-4
- *CHEMICAL ABSTRACTS,* 53858-86-9
- *CHEMICAL ABSTRACTS,* 8047-67-4
- *CHEMICAL ABSTRACTS,* 34089-81-1
- **D.S. KUDASHEVA et al.** Structure of Carbohydrate-bound Polynuclear Oxyhydroxide Nanoparticles in Parenteral Formulation. *J. Inorg. Biochem.,* 2004, vol. 98, 1757-1769
- **I. ERNI et al.** Chemical Characterization of Iron(III) Hydroxide-Dextrin Complexes. *Arzneim.-Forsch./Drug Res.,* 1984, vol. 34 (II), 1555-1559

- **F. FUNK et al.** Physical and Chemical Characterization of Therapeutic Iron Containing Materials. *Hyperfine Interactions,* 2001, vol. 136, 73-95
- **E. LONDON.** The Molecular Formula and Proposed Structure of the Iron-Dextran Complex, IMFERON. *J. Pharm. Sci.,* 2004, vol. 93, 1838-1846
- **A. JOHN.** Neue Möglichkeiten der Eisenversorgung neugeborener Ferkel unter Beachtung biochemischer Aspekte'' [Novel possibilities of supplying iron to new born piglets, taking account of biochemical aspects], Trächtigkeit und Geburt beim Schwein [Pregnancy and birth in pigs. *8th Bernburger Biotechnology Workshop,* 2002, 89-94
- **E.LONDON.** The molecular formula and proposed structure of the iron-dextran complex, Imferon. *J. Pharm. Sci,* 2004, vol. 93 (7), 1838-1845
- **B.LÖNNERDAL.** Calcium and Iron Adsorption - Mechanisms and Public Health Relevance. *Int. J. Vitam. Nutr. Res.,* 2010, vol. 80 (4-5), 293-299
- **M.MIRANDA et al.** Reducing Iron Deficiency Anemia in Bolivian School Children: Calcium and Iron Combined Versus Iron Supplementation Alone. *Nutrition,* 2014, vol. 30, 771-775
- Recommendations of the SEC (Oncology&Haematology), 60th meeting. Akuementis Healthcare Ltd, 12 September 2007